# EUROPEAN PATENT APPLICATION

(11) **EP 3 338 801 A1**
(43) Date of publication of application: **27.06.2018**
(21) Application number: 18153990.9
(22) Date of filing: 09.02.2015
(51) Int. Cl.: A61K 39/395, A61P 35/00, A61K 31/00, C07K 16/28, A61K 39/00

(54) **THERAPEUTIC AGENT FOR SOLID CANCER**

(30) Priority: 21.02.2014 JP 2014032241; 11.06.2014 JP 2014120300; 03.09.2014 JP 2014178953
(62) Divisional of application: 15751324.3
(71) Applicant: Idac Theranostics, Inc., Tokyo 113-0033 (JP); The University Of Tokyo, Tokyo 113-8654 (JP)
(72) Inventor: ITO, Satoru, Bunkyo-ku, Tokyo 113-0033 (JP); YOKOCHI, Shoji, Bunkyo-ku, Tokyo 113-0033 (JP); MATSUSHIMA, Kouji, Bunkyo-ku, Tokyo 113-8654 (JP); UEHA, Satoshi, Bunkyo-ku, Tokyo 113-8654 (JP); ISHIWATA, Yoshiro, Bunkyo-ku, Tokyo 113-0033 (JP)
(74) Representative: Harrison, Susan Joan

(57) **Abstract**

Disclosed is a novel means effective for treatment, metastasis-inhibition, and recurrence-inhibition of human solid cancer. A therapeutic agent for solid cancer according to the present invention comprises as an effective ingredient an anti-CD4 antibody having a high cytotoxic activity, or an anti-CD4 antibody or antigen-binding fragment thereof which antibody or fragment comprises a cytotoxic component bound thereto in combination with an antagonistic anti-LAG-3 antibody. Said anti-CD4 antibody is a human-type chimeric antibody, humanized antibody or human antibody against human CD4. The therapeutic agent of the present invention produces still higher effects by combined use with antagonist or agonist against immune checkpoint molecule, small molecule anticancer agents, or the like. The therapeutic agent is also effective in suppressing recurrence and metastasis of solid cancer.

## Description

### TECHNICAL FIELD

The present invention relates to a therapeutic agent for solid cancer.

### BACKGROUND ART

Recently, cancer immunotherapy is often reported in the news. In 2013, this news has filled the pages in American Society of Clinical Oncology. Thanks to the progress of genome analysis, effects of drugs in individual patients can now be clearly discussed in relation to oncogene abnormalities. However, in spite of such linking between gene abnormalities and effects of drugs, there still remains a problem: what therapeutic strategies can be proposed for patients to whom such links are not applicable? In cancer immunotherapy, gene mutations involved in carcinogenesis do not show direct correlation with effects of drugs. Similarly, however, it is becoming clear that cancer immunotherapy is not necessarily effective for all patients. Thus, research and development of measures for increasing their effectiveness is an urgent task.

One of such measures is to use several drugs in combination to obtain a synergistic effect. It has already been reported that a combination therapy with an anti-CTLA-4 antibody and an anti-PD-1 antibody resulted in an increased effectiveness without causing serious combined side effects (Non-patent Document 1). Since both CTLA-4 and PD-1 molecules are understood as molecules that act at the immune checkpoints, antibodies against these molecules are recognized as immune checkpoint inhibitors (Non-patent Document 2).

Also in the field of carcinostatic drugs, the advent of molecular-targeted drugs has allowed development of anticancer drugs having relatively less side effects. However, negative actions on the body still cannot be avoided. In spite of recent progress in research and development of peptide cancer vaccines, which are part of immunotherapy, those vaccines are not so effective (i.e. the frequency of patients showing effectiveness of the vaccines is low), and therefore the vaccines are not drawing much attention at present. More recently, antibody drugs (for example, Herceptin, an antibody against the molecule called her2/neu) have become available, and they attracted attention as non-synthetic molecular-targeted drugs. However, their problems such as recurrence and drug resistance are also becoming apparent. After the advent of such binding-inhibitory antibodies, patients placed their hope on the effectiveness of such antibodies, but the antibodies have showed only slight survival benefits so far.

Under such circumstances, immune checkpoint inhibitors have appeared as antibody drugs. For example, although antibodies targeting CTLA-4 (molecule expressed on the regulatory T-cell subset) do not have very high tumor regression effects on melanoma, which is known to be a cancer having relatively strong immunogenicity, these antibodies have been found to allow significant prolongation in terms of the survival rate. Thus, these antibodies are now attracting great attention (Non-patent Document 3). It is understood that they cancel the immunosuppressive state of the cancer to enhance an environment in which cytotoxic immune cells can be active. Another example of such a molecule is the PD-1 molecule. Antibody drugs against this molecule have remarkable effects, and they have been found to be even effective for about 30% of patients with solid cancer (for example, lung cancer, colon cancer) (Non-patent Document 4). However, such antibodies have not necessarily been effective for all cancer patients when used alone.

In view of this, combined use of immunotherapeutic agents having different functions has been attempted. First, combined use of an anti-CD4 antibody with an anti-CD 137 (also called as 4-1BB) antibody was proposed using a mouse model (Non-patent Document 5), and, subsequently, combined use of an anti-PD-1 antibody with an anti-CTLA-4 antibody was reported to be very effective according to clinical test data (Non-patent Document 1). The CD137 molecule has an agonistic activity against immune-checkpoint. The PD-1 molecule and the CTLA-4 molecule have immune-checkpoint inhibitory activities. Taking these into account, it is understood that a clinical advantage can be obtained by combined use of drugs that have immune-checkpoint activities but regulate different signaling systems. Although there is a report suggesting that an anti-CD4 antibody alone has a tumor regression effect (Non-patent Document 6), this report is merely based on a study of a mouse model using a particular cancer whose immunogenicity was enhanced by artificial processing of a cancer cell line. Thus, this report does not demonstrate an effect on spontaneous solid cancer. Mouse anti-CD4 antibodies (for example, the GK1.5 clone) used in mouse models are known to have very high complement-dependent cytotoxicity (CDC activity). However, a human-type or humanized anti-CD4 antibody having such a very high cytotoxic effect against CD4-positive (CD4⁺) cells is not yet developed so far to the stage of clinical application (Non-patent Document 7). All humanized anti-CD4 antibodies that have proceeded to clinical tests in the past were those to be used for blood cancers that may contain CD4-expressing tumor cells, such as malignant lymphoma, and no anti-CD4 antibody drug has been developed for solid cancers.

The effectiveness of low-molecular compound anticancer drugs (carcinostatic drugs), which have been historically used as first-line drugs, has been improved with the development of science, and side effects that had been problematic have been reduced by improvement of the doses and the administration methods. However, the antitumor effect of such carcinostatic drugs obtained by balancing between the effect and the toxicity in such a manner is limited, and those drugs are still not necessarily satisfactory at present as drugs for treatment of cancers, which are diseases whose complete cure is difficult.

### PRIOR ART DOCUMENT(S)

### Non-patent Document(s)

Non-patent Document 1: Wolchok JD et al., The New England journal of medicine. 2013;369:122-33.
Non-patent Document 2: Pardoll DM., Nature reviews Cancer. 2012;12:252-64.
Non-patent Document 3: Prieto PA et al., Clinical cancer research : an official journal of the American Association for Cancer Research. 2012;18:2039-47.
Non-patent Document 4: Hamid O et al., The New England journal of medicine. 2013;369:134-44.
Non-patent Document 5: Choi BK et al., Cancer research. 2007;67:8891-9.
Non-patent Document 6: Yu P et al., The Journal of experimental medicine. 2005;201:779-91.
Non-patent Document 7: Kim YH et al., Blood. 2007;109:4655-62.

### SUMMARY OF THE INVENTION

### PROBLEMS TO BE SOLVED BY THE INVENTION

An object of the present invention is to provide novel therapeutic and prophylactic means effective for human solid cancers.

### MEANS FOR SOLVING THE PROBLEMS

For clinical application of anti-CD4 antibodies to human, human-type or humanized anti-CD4 antibodies given a strong, antibody-dependent or complement-dependent cytotoxicity by certain means have to be taken into consideration. Since such expected anti-CD4 antibodies have a property that allows removal of all CD4⁺ cells, they are capable of not only removing regulatory T-lymphocytes, but also widely removing CD4⁺ immune-system cells infiltrating into cancer tissues. Thus, such antibodies could be expected to have a wide range of combination effects.

The present inventors succeeded in establishment of a human-type or humanized anti-CD4 antibodies capable of widely removing CD4⁺ cells and having a high cytotoxic activity, and intensively carried out a model-mouse-level study aiming at development of means which shows a wide range of effects by combination of antibodies based on the established antibodies, thereby accomplishing the present invention.

That is, the present invention provides a therapeutic agent for solid cancer, comprising as an effective ingredient an anti-CD4 antibody having a high cytotoxic activity, or an anti-CD4 antibody or antigen-binding fragment thereof which antibody or fragment comprises a cytotoxic component bound thereto, wherein said anti-CD4 antibody is a human-type chimeric antibody, humanized antibody or human antibody against human CD4. The present invention also provides a recurrence-suppressing agent for solid cancer, comprising as an effective ingredient an anti-CD4 antibody having a high cytotoxic activity, or an anti-CD4 antibody or antigen-binding fragment thereof which antibody or fragment comprises a cytotoxic component bound thereto, wherein said anti-CD4 antibody is a human-type chimeric antibody, humanized antibody or human antibody against human CD4. The present invention further provides a metastasis-suppressing agent for solid cancer, comprising as an effective ingredient an anti-CD4 antibody having a high cytotoxic activity, or an anti-CD4 antibody or antigen-binding fragment thereof which antibody or fragment comprises a cytotoxic component bound thereto, wherein said anti-CD4 antibody is a human-type chimeric antibody, humanized antibody or human antibody against human CD4. The present invention further provides an agent for enhancing activity of, promoting proliferation of, and/or promoting differentiation of CD8⁺ T cells specific to tumor antigen expressed by solid cancer in a solid cancer patient, and/or recruiting the CD8⁺ T cells specific to said tumor antigen to the tumor site in a solid cancer patient, said agent comprising as an effective ingredient an anti-CD4 antibody having a high cytotoxic activity, or an anti-CD4 antibody or antigen-binding fragment thereof which antibody or fragment comprises a cytotoxic component bound thereto, wherein said anti-CD4 antibody is a human-type chimeric antibody, humanized antibody or human antibody against human CD4. The present invention further provides a method of treating solid cancer, comprising administering an effective amount of an anti-CD4 antibody having a high cytotoxic activity, or an anti-CD4 antibody or antigen-binding fragment thereof which antibody or fragment comprises a cytotoxic component bound thereto, to a patient in need of treating solid cancer, wherein said anti-CD4 antibody is a human-type chimeric antibody, humanized antibody or human antibody against human CD4. The present invention further provides a method of suppressing recurrence of solid cancer, comprising administering an effective amount of an anti-CD4 antibody having a high cytotoxic activity, or an anti-CD4 antibody or antigen-binding fragment thereof which antibody or fragment comprises a cytotoxic component bound thereto, to a patient in need of suppressing recurrence of solid cancer, wherein said anti-CD4 antibody is a human-type chimeric antibody, humanized antibody or human antibody against human CD4. The present invention further provides a method of suppressing metastasis of solid cancer, comprising administering an effective amount of an anti-CD4 antibody having a high cytotoxic activity, or an anti-CD4 antibody or antigen-binding fragment thereof which antibody or fragment comprises a cytotoxic component bound thereto, to a patient in need of suppressing metastasis of solid cancer, wherein said anti-CD4 antibody is a human-type chimeric antibody, humanized antibody or human antibody against human CD4. The present invention further provides a method of enhancing activity of, promoting proliferation of, and/or promoting differentiation of CD8⁺ T cells specific to tumor antigen expressed by solid cancer in a solid cancer patient, and/or recruiting the CD8⁺ T cells specific to said tumor antigen to the tumor site in a solid cancer patient, said method comprising administering an effective amount of an anti-CD4 antibody having a high cytotoxic activity, or an anti-CD4 antibody or antigen-binding fragment thereof which antibody or fragment comprises a cytotoxic component bound thereto, to said patient, wherein said anti-CD4 antibody is a human-type chimeric antibody, humanized antibody or human antibody against human CD4.

### EFFECT OF THE INVENTION

The present invention makes it possible to not only treat solid cancers, but also suppress metastasis and recurrence of solid cancers, by using a human-type or humanized anti-CD4 antibody etc. which can exert a sufficient cytotoxic activity against CD4⁺ cells in a human body. The therapeutic effect of the cytotoxic anti-CD4 antibody against blood cancer is exerted through killing of CD4-expressing tumor cells *per se.* On the other hand, when using the therapeutic agent for solid cancer according to the present invention, the immunocompromised environment is canceled by removal of CD4⁺ cells involving immunosuppression, which leads to enhancement of destruction of cancer cells by CD8⁺ CTL (T cells), thereby achieving its therapeutic effect. Furthermore, metastasis and recurrence of solid cancers can also be prevented by the present invention. Conventionally, in the immunotherapy of solid cancer, it has been thought that removal of regulatory T cells (CD4⁺ CD25⁺ Treg cells) is important and that we just have to remove those cells by using a cytotoxic anti-CD25 antibody. However, it is important to remove a wide range of CD4⁺ cells by an anti-CD4 antibody, and effective therapy of human solid cancer can only be accomplished by establishing a human-type or humanized anti CD4 antibody having a strong cytotoxicity. By using a cytotoxic anti-CD4 antibody in combination with an antagonist or agonist against an immune checkpoint molecule or other cellular immunity-stimulating substance etc., the CTL activity of CD8⁺ T cells can be further enhanced to obtain a synergistic effect. Moreover, when using a cytotoxic anti-CD4 antibody in combination with an anticancer drug as a small molecule chemotherapy, the progression of cancer is weakened by growth inhibition or death of cancer cells, and thus a synergistic effect can also be obtained.

### BRIEF DESCRIPTION OF THE DRAWINGS

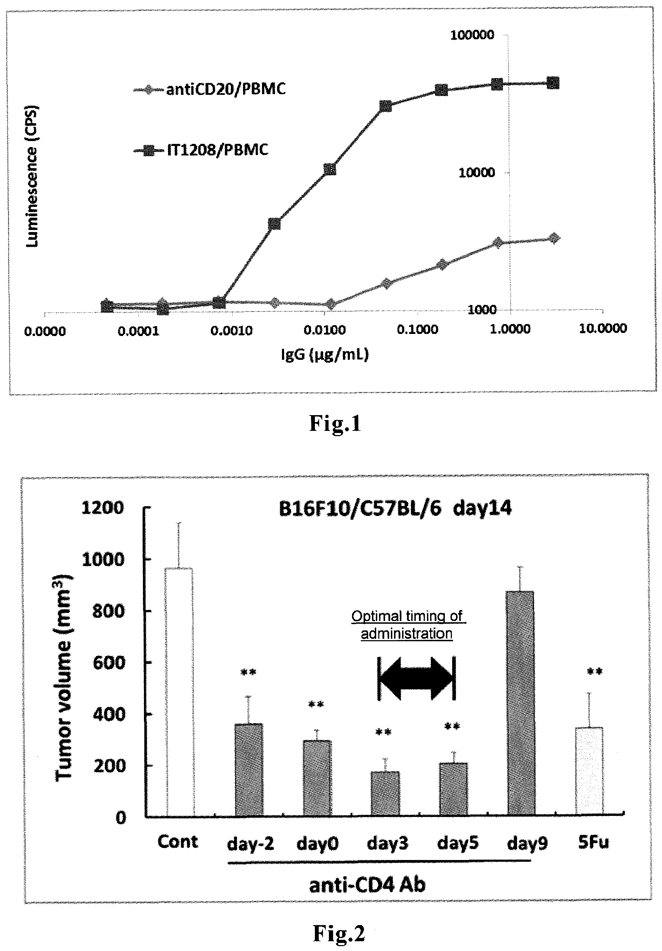

Fig. 1 shows the ADCC activity of an anti-CD4 humanized antibody IT1208 against CD4⁺ cells in human peripheral blood mononuclear cells measured by using a commercially-available assay kit.
Fig. 2 shows the tumor volume in C57BL/6 mice transplanted with a melanoma cell line B16F10. The mice received single administration of anti-CD4 antibody during a period of 2 days before the transplantation to 12 days after the transplantation. The tumor volume was calculated from the solid tumor diameter measured on Day 14. ** indicates a significance level of p<0.01.

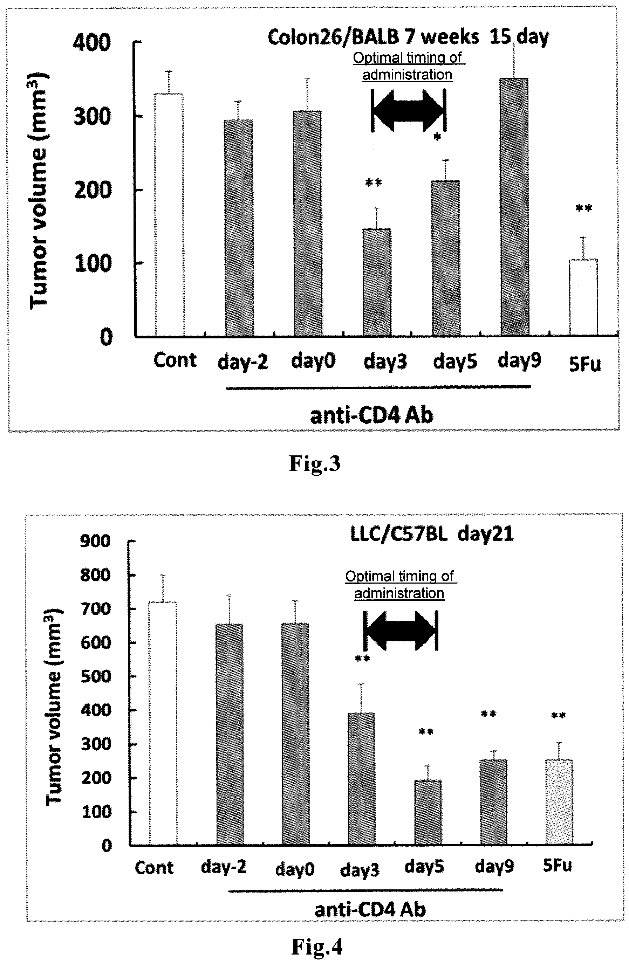

Fig. 3 shows the tumor volume in BALB/c mice transplanted with a colon cancer cell line Colon26. The mice received single administration of anti-CD4 antibody during a period of 2 days before the transplantation to 12 days after the transplantation. The tumor volume was calculated from the solid tumor diameter measured on Day 15. * indicates a significance level of p<0.05. ** indicates a significance level of p<0.01.
Fig. 4 shows the tumor volume in C57BL/6 mice transplanted with a lung cancer cell line LLC. The mice received single administration of anti-CD4 antibody during a period of 2 days before the transplantation to 12 days after the transplantation. The tumor volume was calculated from the solid tumor diameter measured on Day 21. ** indicates a significance level of p<0.01.

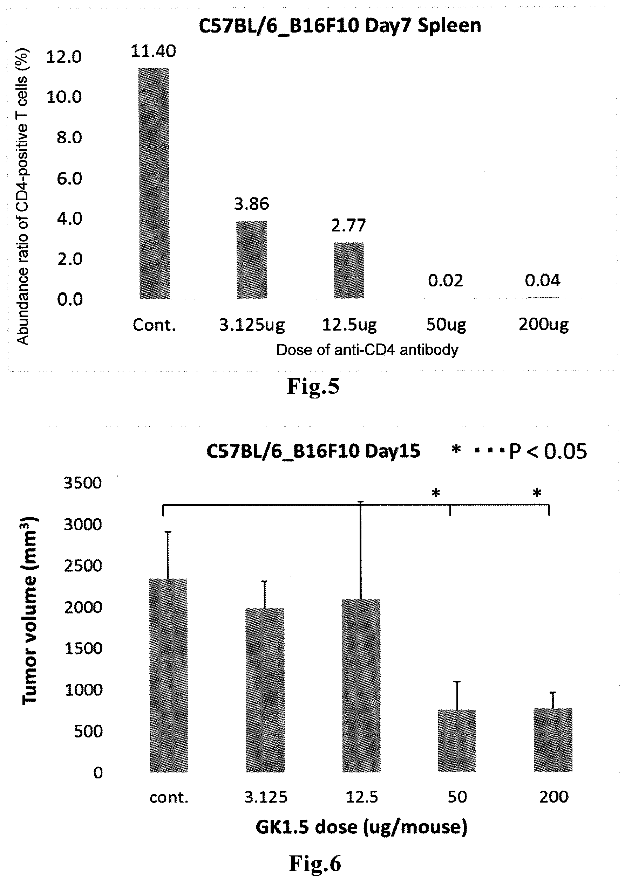

Fig. 5 shows the abundance ratio of CD4⁺ T cells in the lymphocytes extracted from the spleen of C57BL/6 mice transplanted with a melanoma cell line B16F10. The mice received anti-CD4 antibody administration at a various doses. The spleen was excised from the mice on Day 7.
Fig. 6 shows the tumor volume in each group of C57BL/6 mice transplanted with a melanoma cell line B16F10. The mice received an anti-CD4 antibody administration at a various doses. The tumor volume was calculated from the solid tumor diameter measured on Day 15.

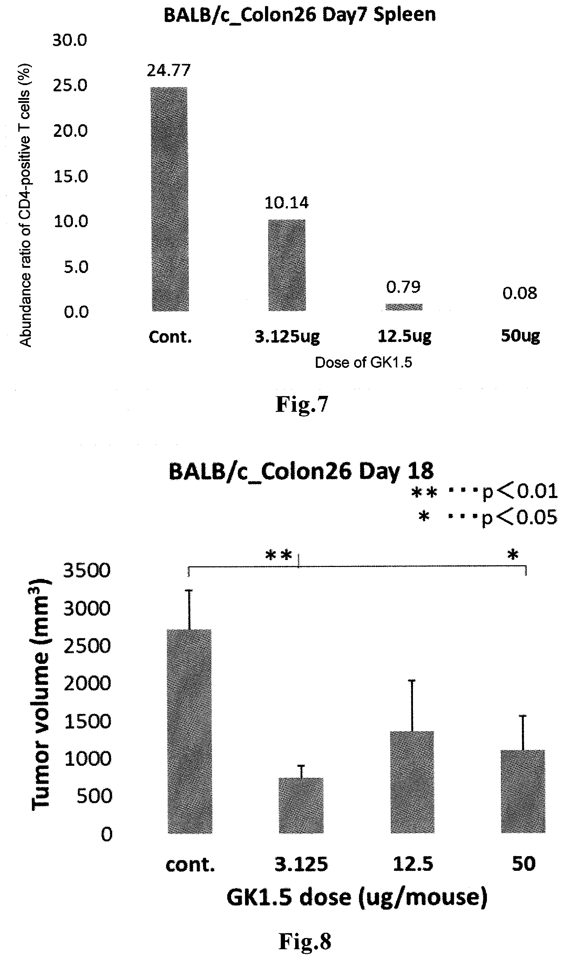

Fig. 7 shows the abundance ratio of CD4⁺ T cells in the lymphocytes extracted from the spleen of BALB/c mice transplanted with a colon cancer cell line Colon26. The mice received anti-CD4 antibody administration at a various doses. The spleen was excised from the mice on Day 7.
Fig. 8 shows the tumor volume in each group of BALB/c mice transplanted with a colon cancer cell line Colon26. The mice received anti-CD4 antibody administration at a various doses. The tumor volume was calculated from the solid tumor diameter measured on Day 18.

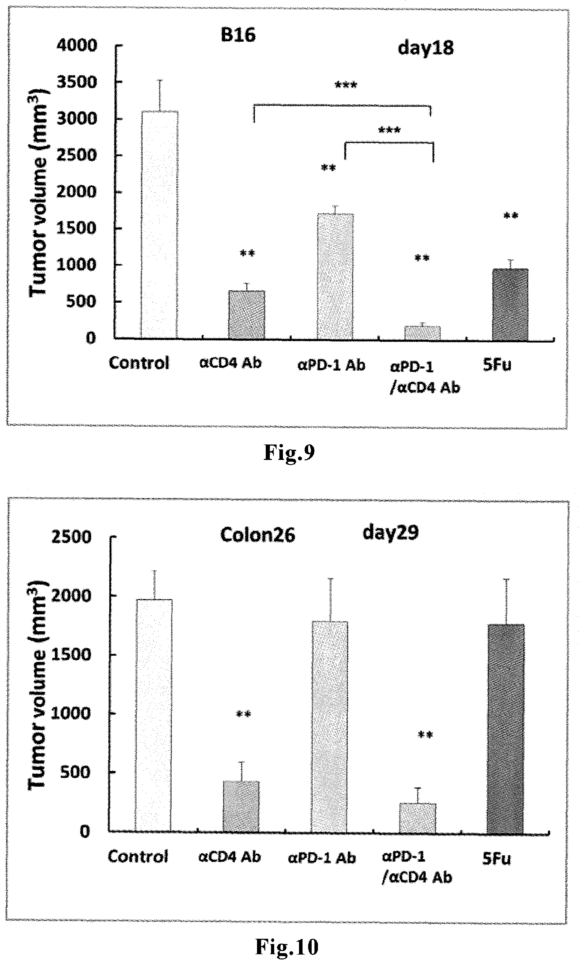

Fig. 9 shows the tumor volume in each group of C57BL/6 mice transplanted with a melanoma cell line B16F10. The tumor volume was calculated from the solid tumor diameter measured on Day 18. The anti-CD4 antibody was administered once, and the anti-PD-1 antibody was administered five times. In the combination group, these were administered in combination. ** indicates a significance level of p<0.01. *** indicates a significance level of p<0.001.
Fig. 10 shows the tumor volume in each group of BALB/c mice transplanted with a colon cancer cell line Colon26. The tumor volume was calculated from the solid tumor diameter measured on Day 29. The anti-CD4 antibody was administered once, and the anti-PD-1 antibody was administered five times. In the combination group, these were administered in combination. ** indicates a significance level of p<0.01.

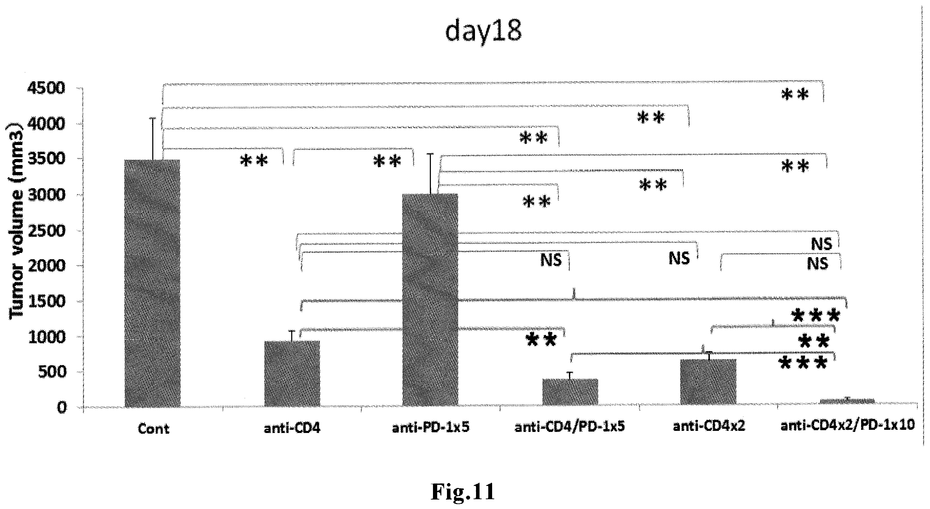

Fig. 11 shows the tumor volume in each group of C57BL/6 mice transplanted with a melanoma cell line B16F10. The tumor volume was calculated from the solid tumor diameter measured on Day 18. The combined administration of anti-CD4 antibody + anti-PD-1 antibody was carried out as a combination of once/five times or twice/ten times.

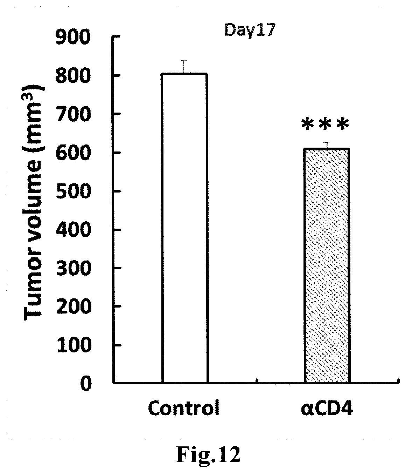

Fig. 12 shows the tumor volume in each group of BALB/c mice transplanted with a breast cancer cell line 4T1. The tumor volume was calculated from the solid tumor diameter measured on Day 17. The anti-CD4 antibody was administered twice. *** indicates a significance level of p<0.001 (t-test).

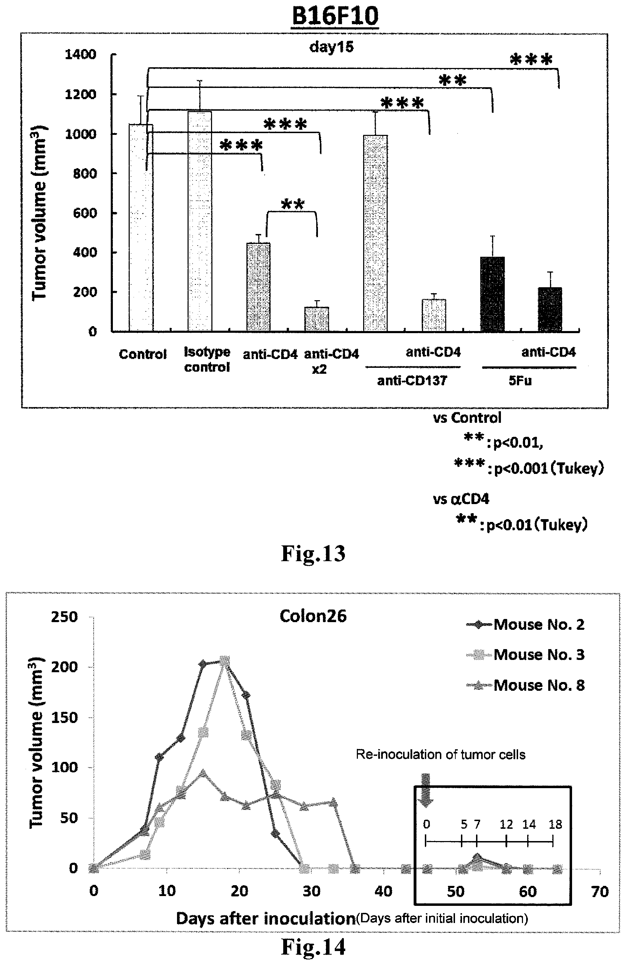

Fig. 13 shows the tumor volume in each group of C57BL/6 mice transplanted with a melanoma cell line B16F10. The tumor volume was calculated from the solid tumor diameter measured on Day 15. In the anti-CD4 antibody alone group, the antibody was administered once or twice. In the anti-CD4 antibody + anti-CD137 antibody combined group, the antibodies were administered once/five times in combination.
Fig. 14 shows the results of tumor size observation. Colon cancer cell line Colon26-transplanted mice which showed complete tumor regression by combined use of anti-CD4 antibody + anti-PD-1 antibody were again transplanted with Colon26 cells, and the tumor size was observed.

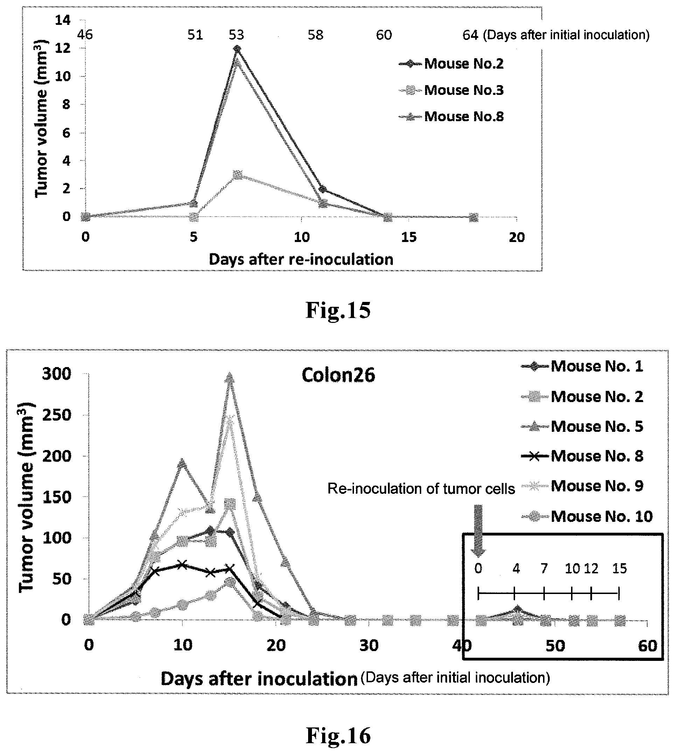

Fig. 15 shows the results of tumor size observation. Colon cancer cell line Colon26-transplanted mice which showed complete tumor regression by combined use of anti-CD4 antibody + anti-PD-1 antibody were again transplanted with Colon26 cells, and the tumor size was observed (a graph showing an enlarged view of the area inside the frame in Fig. 14).
Fig. 16 shows the results of tumor size observation. Colon cancer cell line Colon26-transplanted mice which showed complete tumor regression by combined use of anti-CD4 antibody + anti-PD-Ll antibody were again transplanted with Colon26 cells, and the tumor size was observed.

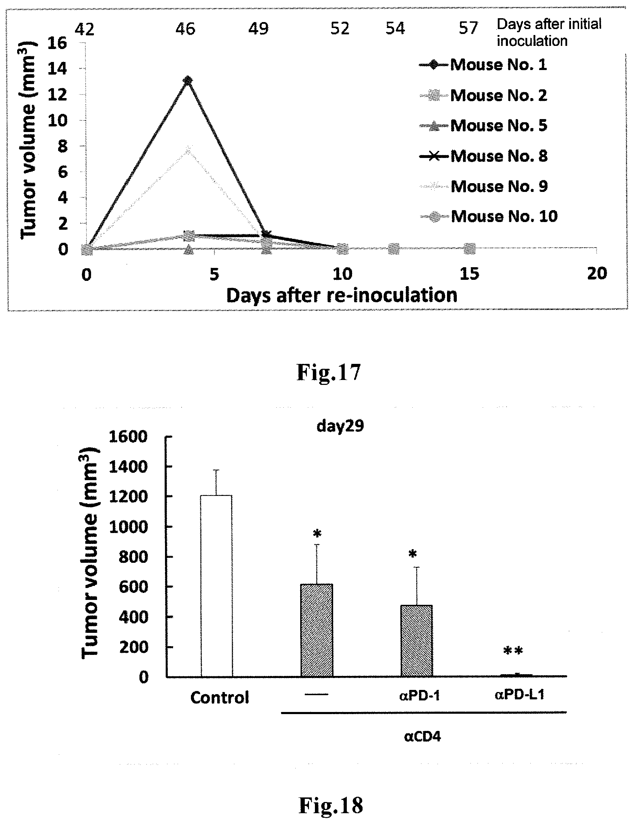

Fig. 17 shows the results of tumor size observation. Colon cancer cell line Colon26-transplanted mice which showed complete tumor regression by combined use of anti-CD4 antibody + anti-PD-Ll antibody were again transplanted with Colon26 cells, and the tumor size was observed (a graph showing an enlarged view of the area inside the frame in Fig. 16).
Fig. 18 shows the tumor volume in each group of BALB/c mice transplanted with a colon cancer cell line Colon26. The tumor volume was calculated from the solid tumor diameter measured on Day 29.

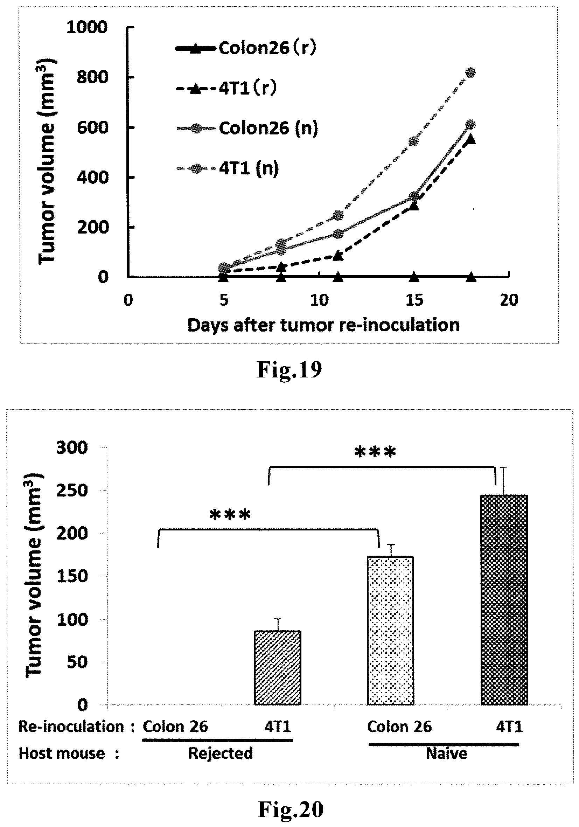

Fig. 19 shows the results of tumor size observation. Colon cancer cell line Colon26-transplanted mice which showed complete tumor regression by combined use of anti-CD4 antibody + anti-PD-1 or anti-PD-Ll antibody were again transplanted with Colon26 cells or a breast cancer cell line 4T1, and the tumor size was observed. (n) represents the tumor size observed in naive mice to which tumor cells were inoculated. The naive mice that had been subjected to neither tumor inoculation nor antibody administration received tumor inoculation.
Fig. 20 shows the average tumor volume on Day 11 (11 days after the re-inoculation) in each mouse group shown in Fig. 19.

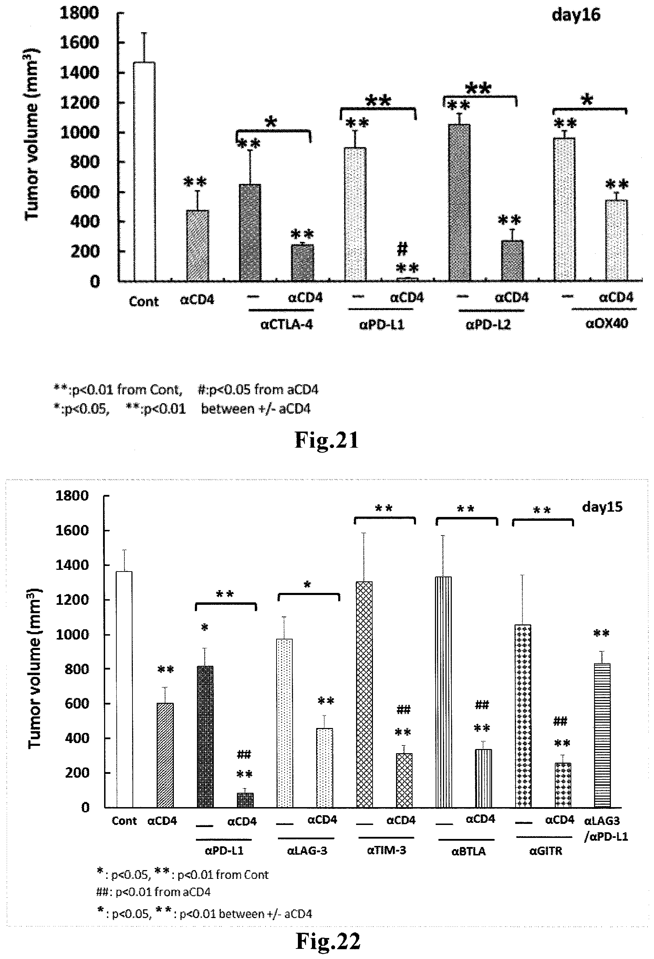

Fig. 21 shows the tumor volume in each group of C57BL/6 mice transplanted with a melanoma cell line B16F10. The tumor volume was calculated from the solid tumor diameter measured on Day 16. Effects of combined use of an anti-CD4 antibody with an anti-PD-Ll antibody, anti-PD-L2 antibody, anti-OX40 antibody, or anti-CTLA-4 antibody were studied.
Fig. 22 shows the tumor volume in each group of C57BL/6 mice transplanted with a melanoma cell line B16F10. The tumor volume was calculated from the solid tumor diameter measured on Day 15. Effects of combined use of an anti-CD4 antibody with an anti-BTLA antibody, anti-GITR antibody, anti-LAG-3 antibody, or anti-TIM-3 antibody were studied.

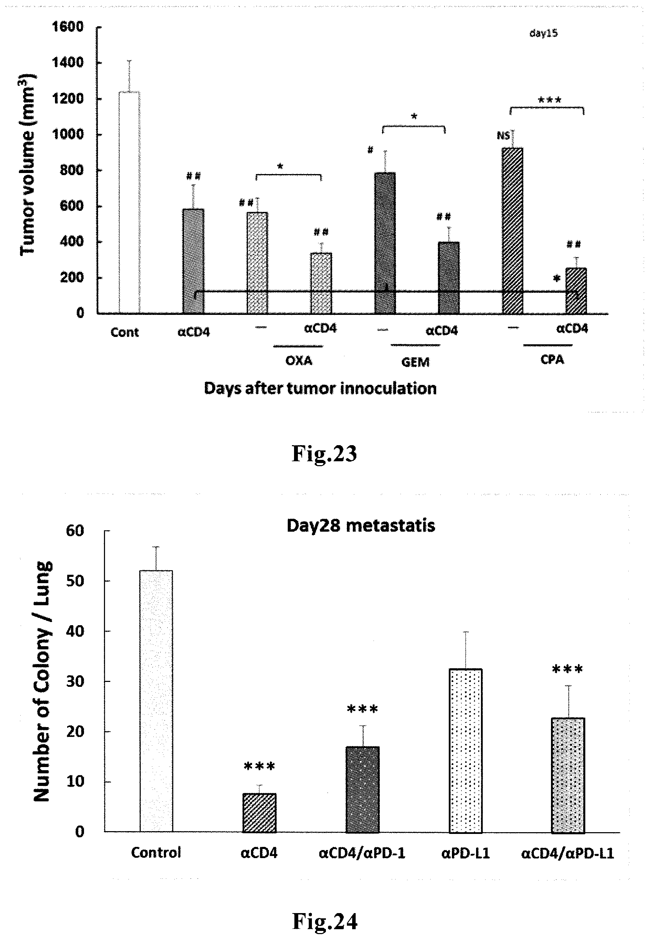

Fig. 23 shows the tumor volume in each group of C57BL/6 mice transplanted with a melanoma cell line B16F10. The tumor volume was calculated from the solid tumor diameter measured on Day 15. The combined administration of anti-CD4 antibody + small molecule anticancer agent was carried out as a combination of twice/four times. ** indicates a significance level of p<0.01.
Fig. 24 shows the number of metastasis to the lungs in each group of BALB/c mice transplanted with the 4T1 cell line. The mice were dissected and their lungs were excised on Day 28 to count the metastasis.

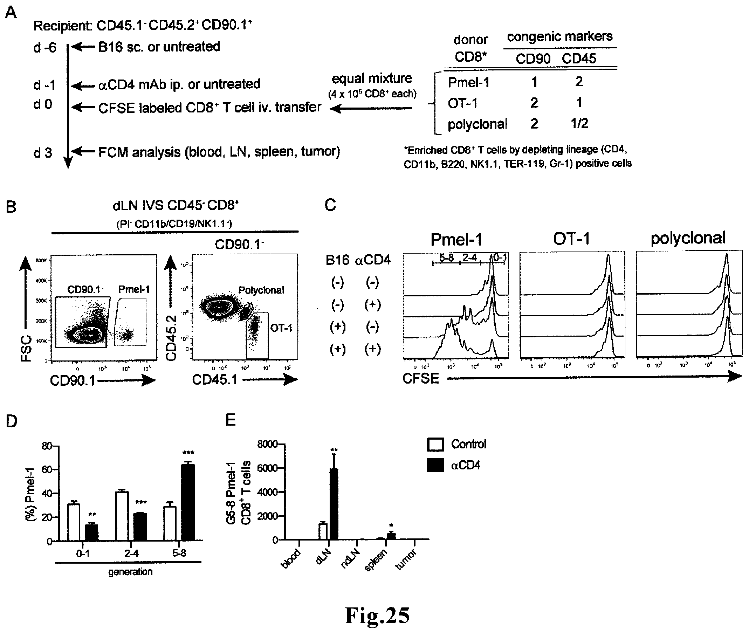

Fig. 25 shows (A) the experimental protocol of Example 9; (B) one example of the results of flow cytometric analysis of tumor-draining lymph node cells in a mouse group receiving adoptive transfer; (C) one example of the results of analysis of the frequency of cell division of lymph node Pmel-1, OT-1, and polyclonal CD8⁺ T cells identified by the development in B, which analysis was carried out using the fluorescence intensity of CFSE as an index; (D) the percentages of cells showing cell division frequency of 0 to 1, 2 to 4, or 5 to 8 in the tumor-draining lymph node Pmel-1 CD8⁺ T cells in the B16F10(+) anti-CD4 antibody(-) group and the B16F10(+) anti-CD4 antibody(+) group; and (E) the numbers of Pmel-1 CD8⁺ T cells showing cell division frequency of 5 to 8 in peripheral blood (blood), tumor-draining lymph node (dLN), non-draining lymph node (ndLN), spleen (spleen), and tumor (tumor) in the B16F10(+) anti-CD4 antibody(-) group and the B16F10(+) anti-CD4 antibody(+) group.

### MODE FOR CARRYING OUT THE INVENTION

Cancers to which the present invention is applied are solid cancers including various cancers except blood cancers (malignant lymphoma, leukemia, multiple myeloma). Typical specific examples include epithelial solid cancers such as lung cancer, breast cancer, gastric cancer, liver cancer, colon cancer, tongue cancer, thyroid cancer, renal cancer, prostate cancer, uterine cancer, cervical cancer, ovarian cancer. However, cancers are not limited as long as they are solid cancers, and the examples also include other solid cancers not belonging to epithelial solid cancers, such as melanoma and glioma. Preferred examples of the solid cancer include, but not limited to, at least one epithelial solid cancer selected from the group consisting of colon cancer, lung cancer, pancreatic cancer, renal cancer, and breast cancer, or at least one epithelial solid cancer selected from the group consisting of colon cancer, lung cancer, pancreatic cancer, and renal cancer, or at least one epithelial solid cancer selected from the group consisting of colon cancer, lung cancer, and breast cancer, or at least one solid cancer selected from melanoma and glioma. The term "treatment of solid cancer" includes both suppression of cancer growth and prolongation of life of cancer patients. The term "treatment of solid cancer" also includes treatment of primary cancer. For example, the therapeutic agent of the present invention may be applied to a patient who developed primary cancer which is different from the primary cancer the patient had first developed, for the purpose of treatment of the second or subsequent primary cancers.

In the present invention, solid cancer is typically spontaneous solid cancer. The spontaneous solid cancer is solid cancer composed of spontaneously occurring cancer cells. The solid cancer is cancer occurring in cells that are not immune cells. CD4, which is expressed in immune cells, is not expressed in solid cancer.

The agent of the present invention can be preferably used for treatment of solid cancer stage I to IV. In mice transplanted with mouse cancer cell line, tumor around the 5th day after the transplantation corresponds to human cancer at stage I, and tumor on the 9th day after B16F10 transplantation and tumor on the 12th day after Colon26 or LLC transplantation correspond to human cancer at stage IV.

The effective ingredient of the therapeutic agent of the present invention is any of the followings. Both of them may be used in combination. In the present specification, the effective ingredients (1) and (2) may be hereinafter collectively referred to as "anti-CD4 component".
(1) An anti-CD4 antibody having a high cytotoxic activity.
(2) An anti-CD4 antibody or antigen-binding fragment thereof, comprising a cytotoxic component bound thereto.

In both cases of (1) and (2), the anti-CD4 antibody is typically an antibody against human CD4, and is a human-type antibody, a humanized antibody (prepared by transplanting the CDR region of a non-human-derived antibody to the corresponding region of a human antibody), or a human antibody (the same antibody as an antibody produced in the body of human, which is prepared using a non-human animal or a human cell line).

The cytotoxic activity antibodies have includes the antibody-dependent cell-mediated cytotoxicity activity (ADCC activity) and the complement-dependent cytotoxicity activity (CDC activity). In cases where the anti-CD4 component belongs to (1) above, the anti-CD4 antibody may have any of the ADCC activity and the CDC activity. It is necessary to use an antibody having a high cytotoxic activity that can exert a sufficiently high ability to kill CD4⁺ cells.

The term "high cytotoxic activity" in the context of the ADCC activity means that an antibody has a higher ADCC activity than the known anti-CD4 antibody 6G5 or CE9.1 that is known to have an ADCC activity, when the ADCC activity against CD4-expressing cells is measured by a known measurement method. In the context of the CDC activity, the term means that an antibody has a stronger CDC activity than the known anti-CD4 antibody OKT4 that is known to have a CDC activity, when the CDC activity against CD4-expressing cells is measured in an experimental system using the same complements by a known measurement method.

Methods for measurement of the ADCC activity and the CDC activity of antibodies are known and described in e.g. Cancer Immunol. Immunother., 36, 373 (1993), and kits therefor are commercially available. Whether a given antibody has a higher cytotoxic activity than known anti-CD4 antibodies or not may be evaluated using such a commercially available kit. A specific example of measurement of the cytotoxic activity using a commercially available kit is described in the Examples below. The level of the ADCC activity of anti-CD4 antibody can also be evaluated by, as described in the Examples below, mixing human peripheral blood mononuclear cells with the anti-CD4 antibody, allowing the reaction to proceed at 37°C for several hours, performing flow cytometry analysis to measure the ratio of CD3⁺ cells to CD8⁺ cells in the reaction solution, and then comparing the obtained measurement value with a measurement value obtained using an anti-CD4 antibody having no ADCC activity or a known anti-CD4 antibody described above.

Preferably, an anti-CD4 antibody having a high cytotoxic activity has an ADCC activity that is 10 times or more, more preferably 100 times or more higher than the ADCC activity of the known anti-CD4 antibody 6G5 and/or CE9.1, or has a CDC activity that is 10 times or more, more preferably 100 times or more higher than the CDC activity of the known anti-CD4 antibody OKT4. As used herein, the term "10 times or more" means, for example, that the minimum antibody concentration at which a given antibody exhibits a cytotoxic activity against a certain amount of cells is one-tenth or less of that of the above-described known antibody. As for the affinity of the anti-CD4 antibody to CD4, the antibody binding activity K_{D} may be about 1 x 10⁻⁹ M or less.

An anti-CD4 antibody having a high cytotoxic activity can be prepared, for example, from a monoclonal anti-CD4 antibody prepared by a known method or from an already established known anti-CD4 antibody, by increasing the cytotoxicity of the antibody by a method known in the art. In cases where an anti-CD4 antibody that specifically recognizes CD4 expressed on the cell surface and has a strong cytotoxicity is known, such an antibody may be used as an effective ingredient of the agent of the present invention. For example, WO 2010/074266 discloses an anti-CD4 antibody having a higher ADCC activity than conventional anti-CD4 antibodies.

A method *per se* of producing a monoclonal antibody is a well-known conventional method in the art. For example, when carrying out the well-known hybridoma method, an anti-CD4 monoclonal antibody can be obtained by immunizing an animal (except human) with a CD4 protein or an appropriate fragment thereof (the extracellular region, e.g., a region from the N-terminus to the 394th amino acid of CD4), collecting antibody-producing cells such as spleen cells or lymphocytes from the immunized animal, fusing the antibody-producing cells with myeloma cells to prepare hybridomas, screening a hybridoma which produces an antibody that binds to CD4 protein, growing the hybridoma, and then collecting an anti-CD4 antibody from the culture supernatant. The gene sequence, amino acid sequence, spatial structure, and the like of CD4 have been deposited in public databases under the accession numbers of, for example, M12807 in GenBank of NCBI. The CD4 protein or an appropriate fragment thereof to be used as an immunogen can be easily prepared based on such sequence information according to well-known genetic engineering methods.

Methods for preparing a chimeric antibody, humanized antibody, or human antibody have been also established as well-known methods in the art. For example, an anti-CD4 human antibody can be prepared by using CDR sequence fragments that ensure CD4 recognition prepared by cassette modification method.

Methods for increasing the cytotoxicity of an antibody are also known, and any of these methods may be used. An example of the known methods is described below.

One method for increasing the ADCC activity is the POTELLIGENT (registered trademark) technology, in which fucose (core fucose) contained in sugar chains present in the Fc region of the antibody is removed (Yamane-Ohnuki N, Satoh M, Production of therapeutic antibodies with controlled fucosylation, MAbs 2009; 1: 230-236.). The enzyme that adds core fucose is encoded by the gene named FucT-8 (Fut-8). Therefore, antibody molecules with enhanced ADCC activity can be obtained by expressing the gene encoding a recombinant antibody in Fut-8 knockout animal cells (Yamane-Ohnuki N, et al., Establishment of FUT8 knockout Chinese hamster ovary cells: an ideal host cell line for producing completely defucosylated antibodies with enhanced antibody-dependent cellular cytotoxicity, Biotechnol Bioeng 2004; 87: 614-622). A method in which fucose substrate donation is blocked is also known, but this method removes all fucose including core fucose, and hence is not specific to core fucose. Thus, the POTELLIGENT (registered trademark) technology described above is more preferred.

Another example of the method for increasing the ADCC activity is a method in which sugar chains present in the Fc region of the antibody is converted. In this method, addition of core fucose is avoided by introduction of GlcNAc in the antenna-type branched sugar chain region by GnT-III gene manipulation (M. Schuster et al., Improved effector functions of a therapeutic monoclonal Lewis Y-specific antibody by glycoform engineering, Cancer Res 2005; 65: 7934-7941). An anti-CD4 antibody having enhanced ADCC activity prepared by such a method may also be used.

A known example of the method for enhancing the CDC activity is the COMPLEGENT (registered trademark) technology, wherein a part of isotype IgG1 is combined with the sequence of isotype IgG3 to increase the CDC activity (Natsume A, In M, Takamura H, et al. Engineered antibodies of IgG1/IgG3 mixed isotype with enhanced cytotoxic activities, Cancer Res. 2008; 68: 3863-3872).

Another known example is the AccretaMab (registered trademark) technology, wherein the POTELLIGENT (registered trademark) technology and the COMPLEGENT (registered trademark) technology described above are employed in combination to strongly increase the cytotoxic activity of an antibody (Natsume A, et al., Improving effector functions of antibodies for cancer treatment: Enhancing ADCC and CDC, Drug Des Devel Ther. 2009; 3: 7-16). An anti-CD4 antibody wherein both ADCC activity and CDC activity are increased by such a method may also be used.

In cases where an anti-CD4 antibody to which a cytotoxic component is bound is used, the antibody does not need to have a high cytotoxic activity, because CD4⁺ cells are injured by the cytotoxic component. An antibody fragment retaining the binding capacity to CD4 (antigen-binding fragment), comprising a cytotoxic component bound thereto may also be used as an effective ingredient of the agent of the present invention.

In the present invention, the cytotoxic component means a substance having an activity to destroy living cells, and includes biological toxic substances, chemical substances, and radioactive substances.

The antigen-binding fragment may be any antibody fragment as long as it retains the binding capacity (antigen-antibody reactivity) to the corresponding antigen of its original antibody. Specific examples of the antigen-binding fragment include, but are not limited to, Fab, F(ab')₂, and scFv. Fab and F(ab')₂ can be obtained, as is well known, by treatment of a monoclonal antibody with a protease such as papain or pepsin. Methods for preparing scFv (single chain fragment of variable region) are also well known. For example, scFv can be obtained by extracting mRNA from a hybridoma prepared as described above, preparing single-stranded cDNA, performing PCR using primers specific to the immunoglobulin H chain and L chain to amplify the immunoglobulin H-chain gene and L-chain gene, linking these using a linker, giving an appropriate restriction enzyme site(s) to the resulting product, introducing the product into a plasmid vector, transforming *E. coli* with the resulting vector to allow expression of scFv, and then recovering the expressed scFv from *E. coli.*

By transiently removing CD4⁺ cells in a cancer patient by administration of anti-CD4 component, the immunocompromised environment in a solid cancer tissue can be canceled, and thus the CTL function of CD8⁺ T cells can be enhanced to effectively remove cancer cells. It has been confirmed that removal of CD4⁺ cells from a cancer-bearing living body results in proliferation of CD8⁺ T cells specific to an antigen of the cancer (tumor antigen). It is thought that an anti-tumor effect of the anti-CD4 component is produced by tumor antigen-specific CD8⁺ T cells whose proliferation, differentiation and/or activity has/have been promoted or enhanced in the immune tissues or tumor tissues, and/or which has been recruited to the tumor site, by administration of anti-CD4 component. A sufficient tumor regression effect is obtained by using the anti-CD4 component alone. It is preferred to use the anti-CD4 component in combination with immune checkpoint inhibitors, various substances having an action to stimulate cellular immunity, immune cell therapy and/or the like, because a still higher anticancer effect (cancer growth inhibitory effect and/or life-prolonging effect) can be obtained.

The term "immune checkpoint molecule" includes both receptors and ligands that function as an immune checkpoint. Immune checkpoints are the immune escape mechanism to prevent the immune system from attacking its own body. Immune checkpoint receptors are present on T cells, and interact with ligands expressed on antigen-presenting cells. T cells recognize an antigen presented on the MHC molecule and are activated to generate an immune reaction, whereas the activation of T cells is controlled by an interaction between immune checkpoint receptor and ligand that occurs in parallel. Immune checkpoint receptors include co-stimulatory receptors and inhibitory receptors, and the T cell activation and the immune reaction are controlled by a balance between both receptors.

Cancer cells express a ligand for an inhibitory immune checkpoint receptor, and escape from attack of cytotoxic T cells utilizing the receptor. Therefore, administration of an antagonist against the inhibitory receptor can prevent cancer cells from utilizing the immune checkpoint mechanism, thereby facilitating killing of cancer cells by CD8⁺ T cells. In addition, administration of an agonist against a co-stimulatory immune checkpoint receptor can enhance the immune reaction, by which killing of cancer cells by CD8⁺ T cells can also be facilitated. In the present invention, at least any of one or more of such antagonists and one or more of such agonists can be preferably used in combination with the anti-CD4 component.

The term "antagonist" includes various substances that interfere with receptor activation induced by binding between receptor and ligand. Examples thereof include substances that interfere with the binding between receptor and ligand by binding to the receptor, and substances that interfere with the binding between receptor and ligand by binding to the ligand.

For example, "an antagonist against an inhibitory immune checkpoint molecule" may be an antagonistic antibody that binds to an inhibitory immune checkpoint molecule (inhibitory receptor or its ligand), a soluble polypeptide that is designed based on an inhibitory immune checkpoint ligand and does not activate the receptor, or a vector capable of expressing the polypeptide, or the like. Examples of the inhibitory immune checkpoint molecule include receptors such as PD-1, CTLA-4, LAG-3, TIM-3, and BTLA, and ligands such as PD-L1 (ligand for PD-1), PD-L2 (ligand for PD-1), CD80 (ligand for CTLA-4), CD86 (ligand for CTLA-4), GAL9 (ligand for TIM-3), and HVEM (ligand for BTLA). Methods of producing an antibody, and methods of producing a polypeptide by chemical synthesis or genetic engineering procedure are well-known conventional methods in the art, and a skilled person can prepare an antagonist against an inhibitory immune checkpoint molecule as described above by conventional methods.

"An agonist against a co-stimulatory immune checkpoint molecule" may be an agonistic antibody that binds to a co-stimulatory immune checkpoint receptor, a soluble polypeptide that is designed based on a co-stimulatory immune checkpoint ligand and has an effect to activate the receptor, or a vector capable of expressing the polypeptide, or the like. Examples of the co-stimulatory immune checkpoint molecule include receptors such as CD137, OX40, and GITR, and ligands such as CD137L (ligand for CD137), OX40L (ligand for OX40), and TNFSF18 (ligand for GITR).

In cases where the anti-CD4 component is used in combination with an antibody, preferred specific examples of the above-described antagonistic antibody include an anti-PD-1 antibody, anti-CTLA-4 antibody, anti-LAG-3 antibody, anti-TIM-3 antibody, and an anti-BTLA antibody, which antibodies bind to a receptor to inhibit binding of a ligand to the receptor, and preferred specific examples of the above-described agonistic antibody include an anti-CD 137 antibody, anti-OX40 antibody, and an anti-GITR antibody, which antibodies bind to a receptor to stimulate a downstream signaling pathway. Preferred specific examples of the antibody also include an anti-PD-Ll antibody, anti-PD-L2 antibody, anti-CD80 antibody, anti-CD86 antibody, anti-GAL9 antibody, and an anti-HVEM antibody, which antibodies bind to a ligand for an inhibitory immune checkpoint receptor to inhibit binding of the ligand to the receptor. The number of the antibody against an immune checkpoint molecule (anti-immune checkpoint antibody) used in combination with the anti-CD4 component is not restricted. One anti-immune checkpoint antibody may be used, or two anti-immune checkpoint antibodies may be used, or three or more anti-immune checkpoint antibodies may be used, in combination with the anti-CD4 component.

Among the above-described antibodies, a preferred antibody that can be preferably used together with the anti-CD4 component may be at least one selected from the group consisting of an antagonistic anti-PD-1 antibody, antagonistic anti-CTLA-4 antibody, antagonistic anti-LAG-3 antibody, antagonistic anti-TIM-3 antibody, antagonistic anti-BTLA antibody, anti-PD-Ll antibody, anti-PD-L2 antibody, agonistic anti-CD137 antibody, agonistic anti-OX40 antibody, and an agonistic anti-GITR antibody; more preferably, at least one selected from the group consisting of an antagonistic anti-PD-1 antibody, antagonistic anti-CTLA-4 antibody, anti-PD-Ll antibody, anti-PD-L2 antibody, agonistic anti-CD137 antibody, and an agonistic anti-OX40 antibody, or at least one selected from the group consisting of an antagonistic anti-LAG-3 antibody, antagonistic anti-TIM-3 antibody, antagonistic anti-BTLA antibody, and an agonistic anti-GITR antibody.

Especially preferred examples include at least one selected from the group consisting of an antagonistic anti-PD-1 antibody, an anti-PD-Ll antibody, and an anti-PD-L2 antibody. A very remarkable anticancer effect can be obtained just by using the anti-CD4 component in combination with at least one selected from an antagonistic anti-PD-1 antibody, an anti-PD-Ll antibody and an anti-PD-L2 antibody, and a still higher therapeutic effect can be obtained by further combining therewith one or more of other immune checkpoint antagonists or agonists or the like (preferred examples include an agonistic anti-CD137 antibody, an agonistic anti-OX40 antibody, an antagonistic anti-CTLA-4 antibody and the like).

Especially preferred examples of the antibody used in combination with the anti-CD4 component also include an antagonistic anti-CTLA-4 antibody. An antagonistic anti-CTLA-4 antibody only may be used in combination with the anti-CD4 component, or one or more of other immune checkpoint antagonists or agonists or the like (preferred examples include an antagonistic anti-PD-1 antibody, an anti-PD-L1 antibody, an anti-PD-L2 antibody, an agonistic anti-CD 137 antibody, an agonistic anti-OX40 antibody and the like) may be further combined with the above, by which a still higher therapeutic effect can be obtained

Especially preferred examples of the antibody used in combination with the anti-CD4 component still further include an antagonistic anti-CD137 antibody. An agonistic anti-CD137 antibody only may be used in combination with the anti-CD4 component, or one or more of other immune checkpoint antagonists or agonists or the like (preferred examples include an antagonistic anti-PD-1 antibody, an anti-PD-L1 antibody, an anti-PD-L2 antibody, an antagonistic anti-CTLA-4 antibody and the like) may be further combined with the above, by which a still higher therapeutic effect can be obtained.

Antibodies against some of immune checkpoints have already been developed, and such known antibodies can also be used. Specific examples of the preferred combination of antibodies include a combination of three components: the anti-CD4 component, an antagonistic anti-PD-1 antibody and an antagonistic anti-CTLA-4 antibody; and a combination of three components: the anti-CD4 component, an anti-PD-L1 antibody and an antagonistic anti-CTLA-4 antibody, but a combination that can be used in the present invention is not limited thereto.

Examples of other substances that can be used in combination with the anti-CD4 component include substances having an action to stimulate cellular immunity or activate NK (natural killer) cells, such as IFN-α/β, IL-12, GM-CSF, and various chemokines (e.g. CCL10, CCL5, RANTES, MIP-1). Combined use of these substances with the anti-CD4 component can further facilitate destruction of cancer cells by the immune system.

Immune cell therapy is a therapeutic method to attack cancer cells using autologous immune cells. Immune cells are taken out of blood or cancer tissue collected or removed from a cancer patient, and cultured *in vitro* to proliferate and activate them. The immune cells are then recovered and administered to the same patient to attack cancer cells in the patient body. Immune cell therapy that can be used in combination with the anti-CD4 component is not limited, and any of known cell therapies conventionally used to treat cancer may be used. Examples of the immune cell therapy include, but are not limited to, TIL therapy in which lymphocytes present in a tumor tissue (tumor-infiltrating lymphocytes) are isolated, proliferated and then administered; LAK therapy in which lymphocytes mainly containing NK cells are collected from a patient, proliferated and then administered; CTL therapy in which lymphocytes are stimulated using lymphocytes and cancer cells collected from a patient to proliferate CTLs specific to cancer cells of the patient, and then the CTLs are administered; and T cell (chimeric antigen receptor; CAR-T) transfer therapy in which T cells produced by genetic modification are transferred. A still higher therapeutic effect can also be obtained by combined use of the agent of the present invention and immune cell therapy.

Further examples of other substances that can be used in combination with the anti-CD4 component include small molecule anticancer agents. A small molecule anticancer agent is an anticancer agent comprising a low-molecular compound as an effective ingredient. The terms "low-molecular compound" and "small molecule drug" in the medical field refer to a chemical substance having a molecular weight of about 1000 to 1500 or below (about several hundreds in general), and a pharmaceutical comprising such a chemical substance as an effective ingredient, which is different from an antibody medicine and nucleic acid medicine. These terms have the same meanings as the above when used in the present invention. Small molecule anticancer agents that can be used in combination with the anti-CD4 component are not restricted. Specific examples of known small molecule anticancer agents include various kinase inhibitors, for example, inhibitors of various tyrosine kinases such as EGFR, Her2, ALK, MET, JAK and the like, including Gefitinib, Erlotinib and Tivantinib; BRAF kinase inhibitors including Vemurafenib and Dabrafenib; and MEK inhibitors including Trametinib. Other specific examples include platinum-based drugs (DNA synthesis inhibition) such as cisplatin, carboplatin and oxaliplatin; pyrimidine-based agents (DNA synthesis inhibition) such as fluorouracil and gemcitabine; camptothecin-based agents (DNA synthesis inhibition) such as irinotecan and topotecan; epipodophyllotoxin-based agents (DNA synthesis inhibition) such as etoposide; vinca alkaloid-based agents (cell division inhibition) such as vinblastine, vincristine, vindesine and vinorelbine; anthracyclines (DNA synthesis inhibition) such as doxorubicin, epirubicin and pirarubicin; taxanes (apoptosis inducer) such as paclitaxel and docetaxel; alkylating agents (DNA synthesis inhibition) such as cyclophosphamide and ifosfamide, and the like. The CD4 component can be used in combination with one or more of small molecule anticancer agents as described above.

The term "combined use" of certain effective ingredients or drugs, or the term "used in combination" means that a plurality of effective ingredients are administered concurrently, sequentially, or separately, to a patient. A plurality of effective ingredients to be used in combination may be provided as separate formulations. In cases where they are administered concurrently, the plurality of effective ingredients may be contained in a single formulation.

The administration route of the anti-CD4 component may be oral or parenteral, and parenteral administration such as intramuscular administration, subcutaneous administration, intravenous administration, or intraarterial administration is preferred. The anti-CD4 component may be administered locally to solid cancer tissue or the vicinity of solid cancer tissue, or may be administered to a regional lymph node in the vicinity of solid cancer, and systemic administration is preferred. The above-described administration routes are also applied to other substances used in combination with the anti-CD4 component.

The anti-CD4 component may be administered at any dose as long as it is effective for therapy of solid cancer to be treated. The effective dose is appropriately selected depending on tumor size, symptoms, age and body weight of the patient, and the like. The dose of the anti-CD4 component may be, but not limited to, about 0.001 mg/kg to 1000 mg/kg, e.g., about 0.01 mg/kg to 100 mg/kg, in terms of the weight of the effective ingredient per day per 1 kg body weight of the patient. The above-described dose may be given to a patient once or dividedly in a few or several times in a day. During the treatment period, the anti-CD4 component may be administered once, or daily for a few or several days, or may be administered multiple times every few or several days, every few or several weeks, or every few or several months.

The dose of the antagonist against the immune checkpoint molecule is also appropriately selected depending on tumor size, symptoms and the like. Usulally, a desirable effect is obtained by increasing the total dose and the frequency of administration of the antagonist more than those of the anti-CD4 component. In cases where an antibody against the immune checkpoint molecule is used as an antagonist, the antibody may be given to a patient at a dose of 1/5 to 5 times the dose of anti-CD4 component per single administration, and at a frequency of 3 to 10 times or more the frequency of administration of anti-CD4 component. Administration of the antagonist may be continued long-term. In cases where single administration of anti-CD4 component and a few or several administrations of antagonist are used in combination, the administration of antagonist can be started before, at the same time as, or after the administration of anti-CD4 component. When an agonist against the immune checkpoint molecule is used in combination with anti-CD4 component, the dose etc. of the agonist may be the same as those of the antagonist.

Other substances and therapies that may be used in combination with anti-CD4 component may be used in the same manner as when they are used alone in cancer therapy. It is also possible to reduce the dose, the frequency of administration, the dosing period, etc. of drugs, since an increased effect is obtained thanks to combined use with anti-CD4 component.

The anti-CD4 component and other substances that may be used in combination therewith can be formulated by appropriately mixing with additives such as pharmaceutically acceptable carriers, diluents, and/or excipients that are suitable for the administration route employed. Examples of the formulation include oral preparations such as tablets, capsules, granules, powders and syrups; and parenteral preparations such as inhalants, injection solutions, suppositories and solutions. Formulation methods and additives that may be used are well-known in the field of formulation of pharmaceuticals, and any of the methods and additives may be used.

By administration of anti-CD4 component, not only a therapeutic effect on solid cancer but also an inhibitory effect on metastasis and recurrence of solid cancer can be obtained. It is confirmed in the Examples described below that mice whose solid tumor has been completely regressed by combined use of an anti-CD4 antibody having a high cytotoxic activity and anti-PD-1 antibody show suppression of growth of the same kind of solid tumor cells transplanted thereto again. This result indicates that the agent according to the present invention has a preventive effect on recurrence and metastasis of solid cancer. Thus, the therapeutic agent according to the present invention can also be used as a metastasis-suppressing agent and a recurrence-suppressing agent for solid cancer. Similarly to the treatment of solid cancer, combined use of the anti-CD4 component with an antagonist and/or agonist against immune checkpoint molecule and/or the like can produce a higher suppressing effect on metastasis and recurrence. The agent according to the present invention can also be used as an agent for enhancing activity of, promoting proliferation of, and/or promoting differentiation of CD8⁺ T cells specific to an antigen (tumor antigen) expressed by solid cancer in a solid cancer patient, and/or recruiting the CD8⁺ T cells specific to said tumor antigen to the tumor site in a solid cancer patient.

### EXAMPLES

The present invention is described below by way of Examples more concretely. However, the present invention is not limited to the Examples described below.

### Example 1: Preparation of Anti-CD4 Antibody Having ADCC Activity

According to the method described in WO 2010/074266, an anti-human CD4 humanized antibody IT1208 having enhanced ADCC activity (wherein HV2 and LV0 described in WO 2010/074266 are contained as the variable region; subtype, IgG1) was prepared. The antibody binding activity as measured using Biacore T100 was K_{D} (nM) < 0.009, which indicates high binding activity.

Measurement of the ADCC activity of IT1208 was carried out under the following conditions, according to the protocol for an ADCC activity assay kit sold by Promega. After gently mixing 12,500 PBMCs derived from a healthy individual, anti-CD4mAb (IT1208), and 75,000 ADCC Bioassay Effector cells contained in the Promega kit, the cells were cultured in a CO₂ incubator at 37°C for 6 hours. The luminescent reagent Bio-Glo reagent was added to the culture, and culturing was then continued at room temperature for 20 minutes, followed by measuring chemiluminescence using a luminescence plate reader.

The results are shown in Fig. 1. IT1208 showed ADCC activity at 1 nM or more, and the activity then increased concentration-dependently to reach the maximum value at 50 nM. In the cases of Rituximab (antiCD20), which was used as a control antibody, the concentration at which the ADCC activity began to be found was 10 nM or more, and the concentration at which the maximum value was achieved was 1 µM or more.

### Example 2-1: Action of Anti-CD4 Antibody against Mouse Solid Cancer Model - Study on Timing of Administration

A mouse melanoma cell line B16F10 (5×10⁵ cells/mouse) was subcutaneously transplanted into the right abdomen of C57BL/6 mice (female, 7 weeks old, n=8); a mouse colon cancer cell line Colon 26 (2×10⁵ cells/mouse) was subcutaneously transplanted into the right abdomen of BALB/c mice (male, 7 weeks old, n=8); and a mouse lung cancer cell line LLC (5×10⁵ cells/mouse) was subcutaneously transplanted into the right abdomen of C57BL/6 mice (female, 7 weeks old, n=8). On Day -2 (two days before the cancer cell transplantation), Day 0 (=day of cancer cell transplantation), Day 3, Day 5, Day 9, or Day 12, 0.2 mg of an anti-CD4 antibody (GK1.5; an antibody known to be capable of causing depletion of CD4+ cells in the mouse body by the CDC activity; manufactured by BioXcell) was intraperitoneally administered at one time. The solid tumor diameter was measured on Day 14, and the tumor volume was calculated by short diameter × short diameter × long diameter × π / 6.

The results from the C57BL/6 mice transplanted with the melanoma cell line B16F10 are shown in Fig. 2; the results from the BALB/c mice transplanted with the colon cancer cell line Colon 26 are shown in Fig. 3; and the results from the C57BL/6 mice transplanted with the lung cancer cell line LLC are shown in Fig. 4. From these results, it was found that the optimal timing of the single administration of the anti-CD4 antibody is Day 0 to Day 12, especially Day 3 to Day 5, after the tumor transplantation.

The growth of solid cancers in the mice transplanted with the cancer cells was observed. In the cases of B16F10 melanoma, when the antibody was administered on Day 5 post-transplantation, the solid tumor had a size at which it could be hardly seen. In contrast, in the cases of Colon 26 and LLC, the solid tumor grew to a size at which it could be seen. In the cases of B16F10 melanoma, formation of the solid tumor delayed by about three days compared to the cases of Colon 26 and LLC. However, B16F10 melanoma showed rapid growth thereafter, and deaths occurred at an early stage. In all cancer cell lines, the administration of the anti-CD4 antibody produced a clear difference in the growth on Day 7 and later, after finding of the solid tumor, as compared to the growth in mice to which the antibody was not administered.

### Example 2-2: Relationship between Abundance Ratio of CD4+ T cells and Antitumor Effect

The mouse melanoma cell line B16F10 (5×10⁵ cells/mouse) was subcutaneously transplanted into the right abdomen of C57BL/6 mice (female, 7 weeks old, n=8) (Day 0 = day of cancer cell transplantation). On Day 5, 3.125 µg, 12.5 µg, 50 µg, or 200 µg of the anti-CD4 antibody was administered (negative control group: no antibody was administered).

Two days after the antibody administration (Day 7), spleens were removed from three mice in each group, and the abundance ratio of CD4+ T cells among lymphocytes extracted therefrom was determined. The results are shown in Fig. 5. In the groups in which 3.125 µg or 12.5 µg of the anti-CD4 antibody was administered, 3.86% or 2.77% of CD4+ T cells were remaining, respectively (negative control group: 11.4%). In contrast, in the groups in which 50 µg or 200 µg of the antibody was administered, the ratio was 0.02% or 0.04%, respectively, indicating complete elimination of those cells.

The solid tumor diameter in each mouse was measured, and the tumor volume was calculated (short diameter × short diameter × long diameter × π / 6). Fig. 6 shows the tumor volume on Day 15 in each group. While significant (Dunnett; significance level, p<0.05) tumor-growth inhibitory effects could be observed in the 50µg-administration group and the 200µg-administration group, in which CD4+ T cells were almost completely eliminated, no difference from the negative control group could be confirmed in the 3.125µg-administration group and the 12.5µg-administration group, in which CD4+ T cells were remaining.

The above results suggest that, in cases of cancer types whose antigenicities are considered to be low, a tumor-growth inhibitory effect can be obtained by complete elimination of CD4+ T cells using an anti-CD4 antibody. When an anti-CD4 antibody is practically applied to a solid-cancer patient, the abundance ratio of CD4+ T cells may be investigated using a peripheral-blood sample or the like to monitor whether the dose is appropriate or not.

### Example 2-3: Relationship between Abundance Ratio of CD4+ T cells and Antitumor Effect

A colon cancer cell line Colon 26 (2×10⁵ cells/mouse) was subcutaneously transplanted into the right abdomen of BALB/c mice (male, 7 weeks old, n=8) (Day 0 = day of cancer cell transplantation). On Day 5, 3.125 µg, 12.5 µg, or 50 µg of the anti-CD4 antibody was administered (negative control group: no antibody was administered).

Two days after the antibody administration (Day 7), spleens were removed from three mice in each group, and the abundance ratio of CD4+ T cells among lymphocytes extracted therefrom was determined. The results are shown in Fig. 7. In the group in which 3.125 µg of the anti-CD4 antibody was administered, 10.14% of CD4+ T cells were remaining (negative control: 24.77%). In contrast, in the group in which 12.5 µg of the antibody was administered, the ratio decreased to 0.79%, and, in the group in which 50 µg of the antibody was administered, those cells were completely eliminated (0.08%).

The solid tumor diameter in each mouse was measured, and the tumor volume was calculated (short diameter × short diameter × long diameter × π / 6). Fig. 8 shows the tumor volume on Day 18 in each group. Although 10.14% (corresponding to about 41% relative to the ratio in the negative control group) of CD4+ T cells were remaining in the 3.125µg-admimstration group, a significantly higher tumor-growth inhibitory effect than that in the negative control group could be observed (Dunnett; significance level, p<0.05). Similarly, the 12.5µg-administration group and the 50µg-administration group showed tendencies to inhibit the tumor growth.

The above results suggest that, in cases of cancer types whose antigenicities are considered to be high, a sufficient tumor-growth inhibitory effect can be obtained even without complete elimination of CD4+ T cells using an anti-CD4 antibody. As mentioned above, when an anti-CD4 antibody is practically applied to a solid-cancer patient, the abundance ratio of CD4+ T cells may be investigated using a peripheral-blood sample or the like to monitor whether the dose is appropriate or not.

### Example 3-1: Comparison of Antitumor Effect by Use of Anti-CD4 Antibody Alone with Antitumor Effect by Use of Anti-PD-1 Antibody Alone or Combined Use of Anti-CD4 Antibody + Anti-PD-1 Antibody

The mouse melanoma cell line B16F10 (5×10⁵ cells/mouse) was subcutaneously transplanted into the right abdomen of C57BL/6 mice (female, 7 weeks old), and the mouse colon cancer cell line Colon 26 (2×10⁵ cells/mouse) was subcutaneously transplanted into the right abdomen of BALB/c mice (male, 7 weeks old). Thereafter, antibody administration was carried out as follows (Day 0 = day of cancer cell transplantation).

**[Table 1]**

| Negative control group | No antibody is administered. |
|---|---|
| Anti-CD4 alone group | An anti-CD4 antibody (0.2 mg; GK1.5) is intraperitoneally administered in a single dose on Day 5. |
| Anti-PD-1 alone group | An anti-PD-1 antibody (0.2 mg; J43, antagonistic antibody, manufactured by BioXcell) is intraperitoneally administered daily for five days from Day 4 to Day 8. |
| Anti-CD4 + anti-PD-1 combination group | An anti-CD4 antibody (0.2 mg) is intraperitoneally administered in a single dose on Day 5, and an anti-PD-1 antibody (0.2 mg) is intraperitoneally administered daily for five days from Day 4 to Day 8. |

Fig. 9 shows the tumor volume in each group of C57BL/6 mice transplanted with the B16F10 cell line. The tumor volume was calculated (short diameter × short diameter × long diameter × π / 6) from the solid tumor diameter measured on Day 18.

The anti-CD4 antibody significantly inhibited the growth of the solid tumor of B16 melanoma to 1/5 (21%) relative to that in the control group (Dunnett; significance level, p<0.01). On the other hand, the anti-PD-1 antibody significantly inhibited the growth of the tumor to about half (55%) relative to that in the control group (Dunnett; significance level, p<0.01). Although the total dose of the anti-CD4 antibody was 1/5 of that of the anti-PD-1 antibody, the antitumor action of the anti-CD4 antibody was significantly stronger than that of the anti-PD-1 antibody (Dunnett; significance level, p<0.01).

On the other hand, combined use of the anti-CD4 antibody and the anti-PD-1 antibody almost completely inhibited the growth of the solid tumor of B16 melanoma (to 6% relative to the growth in the control group), with a significant difference in the average value (Dunnett; significance level, p<0.01). Significant differences in the average tumor volume were also found at a significance level of 1% (Dunnett) between the combination group and the anti-CD4 alone group, and between the combination group and the the anti-PD-1 alone group. Thus, a synergistic effect by the combined use of the anti-CD4 antibody and the anti-PD-1 antibody was strongly suggested.

Fig. 10 shows the tumor volume in each group of BALB/c mice transplanted with the Colon26 cell line. The tumor volume was calculated (short diameter × short diameter × long diameter × π / 6) from the solid tumor diameter measured on Day 29.

The anti-CD4 antibody significantly inhibited the growth of the solid tumor of Colon 26 colon cancer to 1/4 (22%) relative to that in the control group (Dunnett; significance level, p<0.01). On the other hand, the anti-PD-1 antibody hardly inhibited the growth, showing no significant difference (to 91% relative to the growth in the control group; Dunnett, NS; t-test, NS). Although the total dose of the anti-CD4 antibody was 1/5 of that of the anti-PD-1 antibody, the antitumor action of the anti-CD4 antibody was significantly stronger than that of the anti-PD-1 antibody (Dunnett; significance level, p<0.01).

On the other hand, combined use of the anti-CD4 antibody and the anti-PD-1 antibody almost completely inhibited the growth of the solid tumor of Colon 26 colon cancer (to 13% relative to the growth in the control group), with a significant difference in the average value (Dunnett; significance level, p<0.01). A difference, although not significant, in the average tumor volume was found between the combination group and the anti-CD4 antibody group, and a significant difference in the average tumor volume was found between the combination group and the anti-PD-1 antibody administration group at a significance level of 1% (Dunnett). Thus, a synergistic effect by the combined use of the anti-CD4 antibody and the anti-PD-1 antibody was strongly suggested.

### Example 3-2: Comparison of Antitumor Effect by Use of Anti-CD4 Antibody Alone with Antitumor Effect by Use of Anti-PD-1 Antibody Alone or Combined Use of Anti-CD4 Antibody + Anti-PD-1 Antibody (Influence of Increased Frequency of Administration)

The mouse melanoma cell line B16F10 (5×10⁵ cells/mouse) was subcutaneously transplanted into the right abdomen of C57BL/6 mice (female, 7 weeks old), and the mouse colon cancer cell line Colon 26 (2×10⁵ cells/mouse) was subcutaneously transplanted into the right abdomen of BALB/c mice (male, 7 weeks old). Thereafter, antibody administration was carried out as follows (Day 0 = day of cancer cell transplantation).

**[Table 2]**

| Negative control group | No antibody is administered. |
|---|---|
| Anti-CD4 alone group | An anti-CD4 antibody (0.2 mg; GK1.5) is intraperitoneally administered in a single dose on Day 5. |
| Anti-PD-1 alone group | An anti-PD-1 antibody (0.2 mg; J43, antagonistic antibody, manufactured by BioXcell) is intraperitoneally administered daily for five days from Day 4 to Day 8. |
| Anti-CD4 + anti-PD-1 (once/five times) combination group | An anti-CD4 antibody (0.2 mg) is intraperitoneally administered in a single dose on Day 5, and an anti-PD-1 antibody (0.2 mg) is intraperitoneally administered daily for five days from Day 4 to Day 8. |
| Anti-CD4 alone twice administration group | An anti-CD4 antibody (0.2 mg; GK1.5) is intraperitoneally administered twice on Day 5 and Day |
| | 9. |
| Anti-PD-1 alone ten times group | An anti-PD-1 antibody (0.2 mg; J43, manufactured by BioXcell) is intraperitoneally administered daily for five days from Day 4 to Day 8 and again daily for five days from Day 14 to Day 18, ten times in total. |
| Anti-CD4 + anti-PD-1 (twice/ten times) combination group | An anti-CD4 antibody (0.2 mg) is intraperitoneally administered twice on Day 5 and Day 9, and an anti-PD-1 antibody (0.2 mg) is intraperitoneally administered daily for five days and again daily for five days from Day 14 to Day 18, ten times in total. |

Fig. 11 shows the tumor volume in each group of C57BL/6 mice transplanted with the B16F10 cell line. The tumor volume was calculated (short diameter × short diameter × long diameter × π / 6) from the solid tumor diameter measured on Day 18.

The anti-CD4 antibody significantly inhibited the growth of the solid tumor of B16 melanoma to 1/4 (27%) relative to that in the control group (Dunnett; significance level, p<0.01). On the other hand, the anti-PD-1 antibody inhibited the growth to 24% relative to that in the control group. Although the total dose of the anti-CD4 antibody was 1/5 of that of the anti-PD-1 antibody, the antitumor action of the anti-CD4 antibody was significantly stronger than that of the anti-PD-1 antibody (Dunnett; significance level, p<0.01). Combined use of the anti-CD4 antibody and the anti-PD-1 antibody mostly inhibited the growth of the solid tumor of B16 melanoma (to 11% relative to the growth in the control group), with a significant difference in the average value (Dunnett; significance level, p<0.01). Significant differences in the average tumor volume were found at a significance level of 1% (t-test) between the once/five-time combination group and the anti-CD4 alone group, and at a significance level of 0.1% (t-test) between the combination group and the anti-PD-1 alone group. Thus, the experimental results in Example 3-1 showing the synergistic effect by the combination of the anti-CD4 antibody and the anti-PD-1 antibody could be reproduced.

In the twice/ten-time combination group, in which the anti-CD4 antibody was administered twice, and the anti-PD-1 antibody was administered ten times, the growth of the solid tumor of B16 melanoma was almost completely inhibited (to 2% relative to the growth in the control group) with an average value significantly different from that in the control group (Dunnett; significance level, p<0.01). Significant differences in the average tumor volume were also found at a significance level of 0.1% (t-test) between the twice/ten-time combination group and the anti-CD4 alone group, and between the twice/ten-time combination group and the once/five-time combination group. Thus, it was strongly suggested that the synergistic effect by the combination of the anti-CD4 antibody and the anti-PD-1 antibody can be enhanced by increasing the frequency of administration.

### Example 3-3: Study of Antitumor Effect by Use of Anti-CD4 Antibody Alone against Mouse Breast Cancer Cell Line 4T1

A mouse breast cancer cell line 4T1 (1×10⁵ cells/mouse) was subcutaneously transplanted into the mammary fat pad of BALB/c mice (female, 7 weeks old), and antibody administration was carried out as follows (Day 0 = day of cancer cell transplantation).

**[Table 3]**

| Negative control group | No antibody is administered. |
|---|---|
| Anti-CD4 antibody administration group | An anti-CD4 antibody (0.2 mg; GK1.5) is intraperitoneally administered twice on Day 5 and Day 9. |

Fig. 12 shows the tumor volume in each group of BALB/c mice transplanted with the 4T1 cell line. The tumor volume was calculated (short diameter × short diameter × long diameter × π / 6) from the solid tumor diameter measured on Day 17. The anti-CD4 antibody significantly inhibited the growth of the 4T1 solid tumor to 76% relative to that in the control group (t-test; significance level, p<0.001). This result suggested that the anti-CD4 antibody also inhibits the tumor growth of the breast cancer cell line 4T1.

### Example 4: Comparison of Antitumor Effect by Use of Anti-CD4 Antibody Alone with Antitumor Effect by Combined Use of Anti-CD4 Antibody + Anti-CD 137 Antibody or Combined Use of Anti-CD4 antibody + 5-FU

The mouse melanoma cell line B16F10 (5×10⁵ cells/mouse) was subcutaneously transplanted into the right abdomen of C57BL/6 mice (female, 7 weeks old), and the mouse colon cancer cell line Colon 26 (2×10⁵ cells/mouse) was subcutaneously transplanted into the right abdomen of BALB/c mice (male, 7 weeks old). Thereafter, antibody administration was carried out as follows (Day 0 = day of cancer cell transplantation).

**[Table 4]**

| Negative control group | No antibody is administered. |
|---|---|
| Anti-CD4 alone group | An anti-CD4 antibody (0.2 mg; GK1.5) is intraperitoneally administered in a single dose on Day 5. |
| Anti-CD4 alone twice administration group | An anti-CD4 antibody (0.2 mg; GK1.5) is intraperitoneally administered twice on Day 5 and Day 9. |
| Anti-CD137 alone group | An anti-CD137 antibody (0.1 mg; 3H3, antagonist antibody, Juntendo University) is intraperitoneally administered daily for five days from Day 5 to Day 9. |
| 5-FU alone group | 5-FU is administered at a dose of 50 mg/kg twice a week for two weeks (Day 5, Day 9, Day 12, and Day 16). |
| Anti-CD4 + anti-CD137 combination group | An anti-CD4 antibody (0.2 mg) is intraperitoneally administered in a single dose on Day 5, and an anti-CD137 antibody (0.1 mg) is intraperitoneally administered daily for five days from Day 5 to Day 9. |
| Anti-CD4 + 5-FU | An anti-CD4 antibody (0.2 mg) is intraperitoneally |
| combination group | administered in a single dose on Day 5, and 5-FU is administered at a dose of 50 mg/kg twice a week for two weeks. |

Fig. 13 shows the tumor volume in each group of C57BL/6 mice transplanted with the B16F10 cell line. The tumor volume was calculated (short diameter × short diameter × long diameter × π / 6) from the solid tumor diameter measured on Day 15.

The anti-CD4 antibody significantly inhibited the growth of the solid tumor of B16 melanoma by half or more of that in the control group (to 43% relative to the growth in the control group) (Dunnett; significance level, p<0.01). The effect of the single administration of the anti-CD4 antibody was equivalent to the effect of the four times administration of 5-FU. The growth inhibitory effect observed in the cases of twice administration of the anti-CD4 antibody was not less than twice as much as that in the cases of single administration of the antibody. It is expected that complete regression of cancer may be possible by three times administration of the anti-CD4 antibody. On the other hand, the anti-CD137 antibody hardly inhibited the growth, showing no significant difference (to 95% relative to the growth in the control group; Dunnett, NS; t-test, NS). Although the total dose of the anti-CD4 antibody was 1/2.5 of that of the anti-CD137 antibody, the antitumor action of the anti-CD4 antibody was significantly stronger than that of the anti-CD137 antibody (Dunnett; significance level, p<0.01).

On the other hand, combined use of the anti-CD4 antibody and the anti-CD137 antibody almost completely inhibited the growth of the solid tumor of B16 melanoma (to 12%), with a significant difference in the average value (Dunnett; significance level, p<0.05). The average tumor volume showed significant differences between the combination group and the anti-CD4 alone group at a significance level of 5% (t-test), and between the combination group and the anti-CD137 alone group at a significance level of 1% (Dunnett). Thus, a synergistic effect by the combined use of the anti-CD4 antibody and the anti-CD 137 antibody was strongly suggested.

The chemotherapeutic agent 5-FU, which has already been clinically widely used, significantly inhibited the growth of the solid tumor of B16 melanoma to 1/3 (36%) relative to that in the control group (Dunnett; significance level, p<0.01). The tumor growth was more strongly inhibited (to 21% relative to the growth in the control group) in the anti-CD4 + 5-FU combination group as compared to the growth in the groups in which they were individually used, although the difference was not significant. Thus, a synergistic effect by the combination was suggested.

### Example 5-1: Antitumor Effect by Combined Use of Anti-CD4 Antibody + Anti-PD-1 Antibody

The mouse colon cancer cell line Colon 26 (2×10⁵ cells/mouse) was subcutaneously transplanted into the right abdomen of BALB/c mice (male, 7 weeks old). Thereafter, antibody administration was carried out as follows (Day 0 = day of cancer cell transplantation).

**[Table 5]**

| Negative control group | No antibody is administered. |
|---|---|
| Anti-CD4 alone group | An anti-CD4 antibody (0.2 mg; GK1.5) is intraperitoneally administered in a single dose on Day 5. |
| Anti-PD-1 alone group | An anti-PD-1 antibody (0.2 mg; J43, antagonistic antibody, manufactured by BioXcell) is intraperitoneally administered daily for five days from Day 4 to Day 8. |
| Anti-CD4 + anti-PD-1 combination group | An anti-CD4 antibody (0.2 mg) is intraperitoneally administered in a single dose on Day 5, and an anti-PD-1 antibody (0.2 mg) is intraperitoneally administered daily |
| | for five days from Day 4 to Day 8. |

After the antibody administration, the solid tumor diameter in each mouse was measured, and the tumor volume was calculated (short diameter × short diameter × long diameter × π / 6). As a result, the combined use of the anti-CD4 antibody and the anti-PD-1 antibody inhibited the tumor growth in all mice on Day 12 and later. On Day 29, the average tumor volume in these mice became 13% relative to that in the control group.

The evaluation result for each mouse was as follows. On Day 18 and later, four out of eight mice showed reduction in their tumor volumes, and, on Day 36, three out of the eight mice showed complete regression of their tumors (Fig. 14).

Ten days after the complete regression of the tumor (Day 46), the three mice which showed the complete regression on Day 36 were further subjected to subcutaneous inoculation, on their ventral side, of Colon 26 cells in a number five times higher (1×10⁶ cells/mouse) than when the cells were initially inoculated.

The results are shown in Fig. 14 and Fig. 15. Five days after the re-inoculation (Day 51), two out of the three mice showed traces of tumors, and, seven days after the re-inoculation (Day 53), tumor masses were found in all three mice. However, according to comparison of their average tumor volume on Day 53 with that of the mice observed 7 days after the initial inoculation (Day 7), the average on Day 53 was 1/7 relative to the average on Day 7 in the control group, and 1/4 relative to the average on Day 7 in the anti-CD4 antibody + anti-PD-1 antibody combination group.

The tumors whose growth was observed after the re-inoculation showed reduction in their volumes 11 days after the re-inoculation (Day 57) in all mice, and no tumors were found any more in all the mice 14 days after the re-inoculation (Day 64), indicating complete rejection of the tumors.

These results suggest that treatment by the anti-CD4 antibody has an effect to protect against recurrent solid cancers in the body, i.e. that recurrence and metastasis of solid cancers can be suppressed by the agent of the present invention.

### Example 5-2: Antitumor Effect by Combined Use of Anti-CD4 Antibody + Anti-PD-L1 Antibody

The mouse colon cancer cell line Colon 26 (2×10⁵ cells/mouse) was subcutaneously transplanted into the right abdomen of BALB/c mice (male, 7 weeks old). Thereafter, antibody administration was carried out as follows (Day 0 = day of cancer cell transplantation).

**[Table 6]**

| Negative control group | No antibody is administered. |
|---|---|
| Anti-CD4 alone group | An anti-CD4 antibody (0.2 mg; GK1.5) is intraperitoneally administered in a single dose on Day 5. |
| Anti-PD-L1 alone group | An anti-PD-L1 antibody (0.2 mg; 10F.9G2, manufactured by BioXcell) is intraperitoneally administered on Day 4, Day 8, Day 14, and Day 18, four times in total. |
| Anti-CD4 + anti-PD-L1 combination group | An anti-CD4 antibody (0.2 mg) is intraperitoneally administered in a single dose on Day 5, and an anti-PD-L1 antibody (0.2 mg) is intraperitoneally administered on Day 4, Day 8, Day 14, and Day 18, four times in total. |

After the antibody administration, the solid tumor diameter in each mouse was measured, and the tumor volume was calculated (short diameter × short diameter × long diameter × π / 6). As a result, the combined use of the anti-CD4 antibody and the anti-PD-Ll antibody inhibited the tumor growth in all mice on Day 10 and later. On Day 21, the average tumor volume in these mice became 2.5% relative to that in the control group.

The evaluation result for each mouse was as follows. On Day 18 and later, nine out of ten mice showed reduction in their tumor volumes, and, on Day 28, six out of the ten mice showed complete regression of their tumors (Fig. 16).

Fourteen days after the complete regression of the tumor (Day 42), the six mice which showed the complete regression on Day 28 were further subjected to subcutaneous inoculation, on their ventral side, of Colon 26 cells in a number five times higher (1×10⁶ cells/mouse) than when the cells were initially inoculated.

The results are shown in Fig. 16 and Fig. 17. Four days after the re-inoculation (Day 46), four out of the six mice showed traces of tumors. However, according to comparison of their average tumor volume on Day 46 with that of the mice observed 7 days after the initial inoculation (Day 7), the average on Day 46 was 1/20 relative to the average on Day 7 in the anti-CD4 antibody + anti-PD-Ll antibody combination group.

The tumors whose growth was observed after the re-inoculation showed reduction in their volumes 7 days after the re-inoculation (Day 49) in all mice, and no tumors were found any more in all the mice 10 days after the re-inoculation (Day 52), indicating complete rejection of the tumors.

These results also suggest, similarly to Example 5-1, that treatment by the anti-CD4 antibody has an effect to protect against recurrent solid cancers in the body, i.e. that recurrence and metastasis of solid cancers can be suppressed by the agent of the present invention.

### Example 5-3: Antitumor Effects by Combined Use of Anti-CD4 Antibody + Anti-PD-1 Antibody, and Combined Use of Anti-CD4 Antibody + Anti-PD-L1 Antibody - -- Cell-line Specificities of Cancer Antigens

The mouse colon cancer cell line Colon 26 (2×10⁵ cells/mouse) was subcutaneously transplanted into the right abdomen of BALB/c mice (male, 7 weeks old). Thereafter, antibody administration was carried out as follows (Day 0 = day of cancer cell transplantation).

**[Table 7]**

| Negative control group | No antibody is administered. |
|---|---|
| Anti-CD4 alone group | An anti-CD4 antibody (0.2 mg; GK1.5) is intraperitoneally administered in a single dose on Day 5. |
| Anti-PD-1 alone group | An anti-PD-1 antibody (0.2 mg; J43, antagonistic antibody, manufactured by BioXcell) is intraperitoneally administered daily for five days from Day 4 to Day 8. |
| Anti-PD-L1 alone group | An anti-PD-1 antibody (0.2 mg; J43, antagonistic antibody, manufactured by BioXcell) is intraperitoneally administered daily for five days from Day 4 to Day 8. |
| Anti-CD4 + anti-PD-1 combination group | An anti-CD4 antibody (0.2 mg) is intraperitoneally administered in a single dose on Day 5, and an anti-PD-1 antibody (0.2 mg) is intraperitoneally administered daily for five days from Day 4 to Day 8. |
| Anti-CD4 + anti-PD-L1 combination group | An anti-CD4 antibody (0.2 mg) is intraperitoneally administered in a single dose on Day 5, and an anti-PD-1 antibody (0.2 mg) is intraperitoneally administered daily for five days from Day 4 to Day 8. |

After the antibody administration, the solid tumor diameter in each mouse was measured, and the tumor volume was calculated (short diameter × short diameter × long diameter × π / 6). As a result, when the anti-CD4 antibody and the anti-PD-1 antibody were used in combination, inhibition of the tumor growth began in all mice on Day 14 and later. On Day 29, the average tumor volume in these mice became 39% relative to that in the control group (Fig. 18). The combined use of the anti-CD4 antibody and the anti-PD-Ll antibody inhibited the tumor growth in all mice on Day 14 and later. On Day 29, the average tumor volume in these mice became 1% relative to that in the control group (Fig. 18).

The evaluation result for each mouse was as follows. In the anti-CD4 + anti-PD-1 combination group, four out of eight mice showed complete regression of their tumors on Day 32. Similarly, in the anti-CD4 + anti-PD-Ll combination group, seven out of eight mice showed reduction in their tumor volumes, and complete regression of the tumors was achieved on Day 26.

Ten mice with complete regression were divided into two groups each composed of five individuals, and, 17 days after the complete regression of the tumor (Day 49), the mice were further subjected to subcutaneous inoculation, on their ventral side, of Colon 26 cells or 4T1 cells in a number five times higher (1×10⁶ cells/mouse) than when the cells were initially inoculated. The tumor growth was then investigated. For comparison, Colon 26 (2×10⁵ cells/mouse) or 4T1 (2×10⁵ cells/mouse) prepared at the same time was subcutaneously transplanted into the right abdomen of normal BALB/c mice (male, 7 weeks old), and the tumor growth was investigated thereafter. The results are shown in Fig. 19 and Fig. 20.

In the BALB/c mice into which Colon 26 (2×10⁵ cells/mouse) or 4T1 (2×10⁵ cells/mouse) was transplanted for the first time (n, Native), both cell lines clearly showed the tumor growth, and it was therefore evident that the growth activities of both cell lines were maintained.

The results on the Colon 26-rejected mice (r, Rejected) were as follows. In the group re-inoculated with Colon 26, which is the colon cancer cell line that had once been rejected, no additional tumor growth was found in all the mice unlike normal mice, indicating complete rejection of the tumor. In contrast, in the group in which the Colon 26-rejected mice were inoculated with the breast cancer cell line 4T1, which is different from the once-rejected Colon 26, development of cancer was found five days after the inoculation (Day 54), and the cancer continuously grew thereafter without being rejected. However, although the breast cancer cell line 4T1 was not rejected in the Colon 26-rejected mice, its tumor growth rate was significantly lower than that in the normal mice to which the cell line was transplanted.

These results suggest that treatment by the anti-CD4 antibody has an effect to protect against recurrent solid cancers in the body, i.e. that recurrence and metastasis of solid cancers can be suppressed by the agent of the present invention. In particular, it was strongly suggested that the agent inhibits the growth of primary cancers developed in other sites, as well as the growth of the same type of cancer.

### Example 6: Antitumor Effects by Combined Use of Anti-CD4 Antibody with Anti-PD-L1 Antibody, Anti-PD-L2 Antibody, Anti-OX40 Antibody, or Anti-CTLA-4 Antibody

The mouse melanoma cell line B16F10 (5×10⁵ cells/mouse) was subcutaneously transplanted into the right abdomen of C57BL/6 mice (female, 7 weeks old, n=8), and antibody administration was carried out as described below (Day 0 = day of cancer cell transplantation).

**[Table 8]**

| Negative control group | No antibody is administered. |
|---|---|
| Anti-CD4 alone group | An anti-CD4 antibody (0.2 mg; GK1.5) is intraperitoneally administered twice on Day 5 and Day 9. |
| Anti-PD-Ll, anti-PD-L2, anti-OX40, or anti-CTLA-4 alone group | An anti-PD-L1 antibody (10F.9G2, manufactured by BioXcell), anti-PD-L2 antibody (TY25, manufactured by BioXcell), anti-OX40 antibody (OX-86, agonist antibody; manufactured by BioXcell), or anti-CTLA-4 antibody (9D9, antagonist antibody; manufactured by BioXcell) is intraperitoneally administered at a dose of 0.2 mg on Day 4, Day 8, Day 14, and Day 18, four times in total. |
| Anti-CD4 + anti-PD-Ll, anti-PD-L2, anti-OX40, or anti-CTLA-4 combination group | An anti-CD4 antibody (0.2 mg) is intraperitoneally administered twice on Day 5 and Day 8, and an anti-PD-L1, anti-PD-L2, anti-OX40, or anti-CTLA-4 antibody is administered at a dose of 0.2 mg on Day 4, Day 8, Day 14, and Day 18, four times in total. |

Fig. 21 shows the tumor volume in each group of C57BL/6 mice transplanted with the B16F10 cell line. The tumor volume was calculated (short diameter × short diameter × long diameter × π / 6) from the solid tumor diameter measured on Day 16.

The anti-CD4 antibody significantly inhibited the growth of the solid tumor of B16 melanoma to about 1/3 relative to that in the control group (Dunnett; significance level, p<0.01). Here, based on observation of the tumor-growth inhibitory effect by the single use of each agent, it is clearly shown that the anti-CD4 antibody has a better inhibitory effect than those of the other anti-immune checkpoint antibodies.

On the other hand, when the anti-PD-L1 antibody, anti-PD-L2 antibody, anti-OX40 antibody, and anti-CTLA-4 antibody were individually administered, significantly stronger inhibition of the growth could be observed relative to the growth in the control group (Dunnett; significance level, p<0.01), although the inhibition was weaker than that by the anti-CD4 antibody. When the anti-CD4 antibody was used in combination with the anti-PD-Ll antibody, anti-PD-L2 antibody, anti-OX40 antibody, or anti-CTLA-4 antibody, the growth of the B16 melanoma solid tumor was more strongly inhibited than in the groups in which the antibodies were individually administered without administration of the anti-CD4 antibody. The averages in the immune checkpoint antibody alone groups were significantly different from the averages in the anti-CD4 combination groups (Dunnett; significance level, p<0.05 or p<0.01). Thus, synergistic effects by the combinations became apparent. In particular, the average tumor volume in the anti-CD4 + anti-PD-Ll combination group was significantly different from that in the anti-CD4 alone group (significance level, 5%; Dunnett). Thus, a remarkable synergistic effect by the combined use of the anti-CD4 antibody and the anti-PD-L1 antibody was shown.

### Example 7: Antitumor Effects by Combined Use of Anti-CD4 Antibody with Anti-BTLA Antibody, Anti-GITR Antibody, Anti-LAG-3 Antibody, or Anti-TIM-3 Antibody

The mouse melanoma cell line B16F10 (5×10⁵ cells/mouse) was subcutaneously transplanted into the right abdomen of C57BL/6 mice (female, 7 weeks old, n=8), and antibody administration was carried out as described below (Day 0 = day of cancer cell transplantation).

**[Table 9]**

| Negative control group | No antibody is administered. |
|---|---|
| Anti-CD4 alone group | An anti-CD4 antibody (0.2 mg; GK1.5) is intraperitoneally administered twice on Day 5 and Day 9. |
| Anti-BTLA, anti-GITR, | An anti-BTLA antibody (6A6, manufactured by |
| anti-LAG-3, or anti-TIM-3 alone group | BioXcell), anti-GITR antibody (DTA-1, agonist antibody; manufactured by BioXcell), anti-LAG-3 antibody (C9B7W, manufactured by BioXcell), or anti-TIM-3 antibody (RMT3-23, manufactured by BioXcell) is intraperitoneally administered at a dose of 0.2 mg on Day4, Day8, Day14, and Day18, four times in total. |
| Anti-CD4 + anti-BTLA, anti-GITR, anti-LAG-3, or anti-TIM-3 combination group | An anti-CD4 antibody (0.2 mg) is intraperitoneally administered twice on Day 5 and Day 8, and an anti-BTLA antibody, anti-GITR antibody, anti-LAG-3 antibody, or an anti-TIM-3 antibody is intraperitoneally administered at a dose of 0.2 mg on Day4, Day8, Day14, and Dayl8, four times in total. |

Fig. 22 shows the tumor volume in each group of C57BL/6 mice transplanted with the B16F10 cell line. The tumor volume was calculated (short diameter × short diameter × long diameter × π / 6) from the solid tumor diameter measured on Day 15.

The anti-CD4 antibody significantly inhibited the growth of the solid tumor of B16 melanoma to about half relative to that in the control group (Dunnett; significance level, p<0.01). Here, based on observation of the tumor-growth inhibitory effect by the use of each agent alone, it is clearly shown that the anti-CD4 antibody has a better inhibitory effect than the other anti-immune checkpoint antibodies when used alone.

On the other hand, when the anti-LAG-3 antibody alone was administered, significantly stronger inhibition of the growth could be observed relative to the growth in the control group (Dunnett; significance level, p<0.05), although the inhibition was weaker than that by the anti-CD4 antibody. When the anti-CD4 antibody was used in combination with the anti-BTLA antibody, anti-GITR antibody, anti-LAG-3 antibody, or anti-TIM-3 antibody, the growth of the B16 melanoma solid tumor was more strongly inhibited than in the groups in which the antibodies were individually administered without administration of the anti-CD4 antibody. The averages in the immune checkpoint antibody alone groups were significantly different from the averages in the anti-CD4 antibody combination groups (Dunnett; significance level, p<0.05 or p<0.01). Thus, synergistic effects by the combinations became apparent. In particular, the average in the anti-CD4 antibody alone group was significantly different from the averages in the groups in which the anti-BTLA antibody, anti-GITR antibody, or anti-TIM-3 antibody was combined (Dunnett; significance level, p<0.01), indicating remarkable synergistic effects.

### Example 7: Antitumor Effect by Combined Use of Anti-CD4 Antibody + Small Molecule Anticancer Agent

The mouse melanoma cell line B16F10 (5×10⁵ cells/mouse) was subcutaneously transplanted into the right abdomen of C57BL/6 mice (female, 7 weeks old), and drug administration was carried out as described below (Day 0 = day of cancer cell transplantation).

**[Table 10]**

| Negative control group | No drug is administered. |
|---|---|
| Anti-CD4 alone group | An anti-CD4 antibody (0.2 mg; GK1.5) is intraperitoneally administered twice on Day 5 and Day 9. |
| Oxaliplatin (OXA, ELPLAT I. V. INFUSION SOLUTION, Yakult) alone group | Oxaliplatin is administered at a dose of 10mg/kg twice a week for two weeks (Day 5, Day 9, Day 12, and Day 16). |
| Anti-CD4 + OXA combination group | An anti-CD4 antibody (0.2 mg) is intraperitoneally administered twice on Day 5 and Day 9, and OXA is administered at a dose of 10 mg/kg twice a week for two weeks. |
| Gemcitabine (GEM, Gemzar Injection; Eli Lilly Japan) alone group | Gemcitabine is administered at a dose of 20 mg/kg twice a week for two weeks (Day 5, Day 9, Day 12, and Day 16). |
| Anti-CD4 + GEM combination group | An anti-CD4 antibody (0.2 mg) is intraperitoneally administered twice on Day 5 |
| | and Day 9, and GEM is administered at a dose of 20 mg/kg twice a week for two weeks. |
| Cyclophosphamide (CPA, Endoxan Injection; Shionogi & Co., Ltd.) alone group | Cyclophosphamide is administered at a dose of 10 mg/kg twice a week for two weeks (Day 5, Day 9, Day 12, and Day 16). |
| Anti-CD4 + CPA combination group | An anti-CD4 antibody (0.2 mg) is intraperitoneally administered twice on Day 5 and Day 9, and OXA is administered at a dose of 10 mg/kg twice a week for two weeks. |

Fig. 23 shows the tumor volume in each group of C57BL/6 mice transplanted with the B16F10 cell line. The tumor volume was calculated (short diameter × short diameter × long diameter × π / 6) from the solid tumor diameter measured on Day 15.

The anti-CD4 antibody significantly inhibited the growth of the solid tumor of B16 melanoma by half or more of that in the control group (to 47% relative to the growth in the control group) (Dunnett; significance level, p<0.01). On the other hand, in the OXA alone group, the growth was inhibited to the same degree as in the anti-CD4 antibody alone group (to 46% relative to the growth in the control group) (Dunnett; significance level, p<0.01). However, weight loss was observed in mice receiving OXA at this dose, indicating occurrence of a side effect. In the GEM alone group, the growth was inhibited to 63% relative to that in the control group, with weaker inhibition than that in the anti-CD4 antibody alone group (Dunnett; significance level, p<0.05). On the other hand, CPA only weakly inhibited the growth (to 75% relative to the growth in the control group), showing no significant difference from the control group.

When the anti-CD4 antibody was used in combination with OXA, GEM, or CPA, the growth of the solid tumor of B16 melanoma was more strongly inhibited than when the anti-CD4 antibody was used alone. In particular, the average in the CPA combination group was significantly different from the average in the anti-CD4 alone group (Dunnett; significance level, p<0.05). In all the combination groups, the effect was remarkably enhanced when compared to the OXA, GEM, or CPA alone groups, and the differences between the averages were significant (Dunnett; OXA: significance level, p<0.05; GEM: significance level, p<0.05; CPA: significance level, p<0.001), strongly suggesting synergistic effects by combined use of the anti-CD4 antibody with the anticancer drugs.

### Example 8: Comparison of Antimetastatic Effect by Use of Anti-CD4 Antibody Alone with Antimetastatic Effect by Combined Use of Anti-CD4 Antibody + Anti-PD-1 Antibody or Combined Use of Anti-CD4 Antibody + Anti-PD-Ll Antibody, against Mouse Breast Cancer Cell Line 4T1

A mouse breast cancer cell line 4T1 (1×10⁵ cells/mouse) was subcutaneously transplanted into the mammary fat pad of BALB/c mice (female, 7 weeks old), and antibody administration was carried out as follows (Day 0 = day of cancer cell transplantation).

**[Table 11]**

| | |
|---|---|
| Negative control group | No antibody is administered. |
| Anti-CD4 alone group | An anti-CD4 antibody (0.2 mg; GK1.5) is intraperitoneally administered twice on Day 5 and Day 9. |
| Anti-PD-L1 alone group | An anti-PD-L1 antibody (0.2 mg; 10F.9G2, manufactured by BioXcell) is intraperitoneally administered on Day4, Day8, Day14, and Day18, four times in total. |
| Anti-CD4 + anti-PD-1 combination group | An anti-CD4 antibody (0.2 mg; GK1.5) is intraperitoneally administered twice on Day 5 and Day 9, and an anti-PD-1 antibody (0.2 mg; J43, manufactured by BioXcell) is intraperitoneally administered on Day4, Day8, Day14, and Day18, four times in total. |
| Anti-CD4 + anti-PD-L1 combination group | An anti-CD4 antibody (0.2 mg; GK1.5) is intraperitoneally administered twice on Day 5 and Day |
| | 9, and an anti-PD-L1 antibody (0.2 mg; 10F.9G2, manufactured by BioXcell) is intraperitoneally administered on Day4, Day8, Day14, and Dayl8, four times in total. |

Fig. 24 shows the number of metastasis to the lungs in each group of BALB/c mice transplanted with the 4T1 cell line. The mice were dissected and their lungs were excised on Day 28 to count the metastasis.

The anti-CD4 antibody significantly reduced the number of metastasis of 4T1 to the lungs to 15% relative to that in the control group (Dunnett; significance level, p<0.001). The anti-CD4 + anti-PD-1 combination group significantly reduced the number of metastasis to 32% relative to that in the control group (Dunnett; significance level, p<0.001).

On the other hand, in the anti-PD-L1 alone group, there was a tendency to inhibit metastasis, but no significant difference was found. However, the anti-CD4 + anti-PD-L1 combination group significantly reduced the number of metastasis to 44% relative to that in the control group (Dunnett; significance level, p<0.001), showing an enhanced antimetastatic effect by the combined use.

### Example 9: Study on Antigen Specificity of CD8⁺ T Cells That Increase in Tumor-Draining Lymph Nodes after Administration of Anti-CD4 Antibody Alone

The mouse melanoma cell line B16F10 (5×10⁵ cells/mouse) was subcutaneously inoculated into the right side of the back of C57BL/6 mice (congenic markers CD45.1⁻ CD45.2⁺ CD90.1⁻ CD90.2⁺), and 0.2 mg of the anti-CD4 antibody (GK1.5) was intraperitoneally administered in a single dose on Day 5. On Day 6, a mixed suspension containing the following three kinds of the same number of CD8⁺ T cells having different antigen specificities, labeled with CFSE using Vybrant (registered trademark) CFDA SE Cell Tracer Kit (Life technologies), was transferred from the tail vein (adoptive transfer).
(1) Pmel-1 CD8⁺ T Cells:
   CD8⁺ T cells prepared from a Pmel-1 mouse, which mouse was prepared by gene transfer of gp100 melanoma antigen-specific MHC class I-restricted TCR (congenic markers: CD45.1⁻ CD45.2⁺ CD90.1⁺ CD90.2⁻).
(2) OT-1 CD8⁺ T Cells:
   CD8⁺ T cells prepared from an OT-1 mouse, which mouse was prepared by gene transfer of ovalbumin-specific MHC class I-restricted TCR (congenic markers: CD45.1⁺ CD45.2⁻ CD90.1⁻ CD90.2⁺).
(3) Polyclonal CD8⁺ T Cells:
   CD8⁺ T cells prepared from a wild-type mouse (congenic markers: CD45.1⁺ CD45.2⁺ CD90.1⁻ CD90.2⁺)

Besides the B16F10(+) anti-CD4 antibody(+) group, a B16F10(-) anti-CD4 antibody(-) group, a B16F10(-) anti-CD4 antibody(+) group, and a B16F10(+) anti-CD4 antibody(-) group were provided as control groups.

Three days after the adoptive transfer, cell suspensions were prepared from the tumor (tumor), tumor-draining brachial lymph node (dLN), non-draining brachial lymph node (ndLN), peripheral blood (blood), and spleen (spleen). The cells were stained with fluorescence-labeled antibodies specific to the surface antigens, and the number of donor CD8+ T cells transferred and the frequency of cell division were analyzed by flow cytometry.

In the mouse groups used in the present experiment, Pmel-1 CD8⁺ T cells in the B16F10(+) group are capable of recognizing the specific antigen. On the other hand, OT-1 and polyclonal CD8⁺ T cells are not capable of recognizing the specific antigen in any of the groups. That is, if the administration of the anti-CD4 antibody promotes the growth response not only for Pmel-1 CD8⁺ T cells in the B16F10(+) group, but also for OT-1 and polyclonal CD8⁺ T cells in any of the groups, the growth response can be understood as an antigen-nonspecific growth response. On the other hand, if the growth response is promoted only for Pmel-1 CD8⁺ T cells in the B16F10(+) group, the growth response can be understood as a tumor antigen-specific growth response.

Fig. 25A shows the experimental protocol.

Fig. 25B shows an example of flow cytometric analysis of the tumor-draining lymph node cells. The IVS-CD45⁻ CD8⁺ fraction was developed according to the expression of CD90.1/FSC to identify CD90.1+ Pmel-1 cells. The CD90.1⁻ fraction was developed according to the expression of CD45.1/CD45.2 to separately identify CD45.1⁻ CD45.2⁺ host CD8⁺ T cells, CD45.1⁺ CD45.2⁺ Polyclonal CD8⁺ T cells, and CD45.1⁺ CD45.2⁺ OT-1 CD8⁺ T cells.

Fig. 25C shows an example of the results of analysis of the frequency of cell division of lymph node Pmel-1, OT-1, and Polyclonal CD8⁺ T cells identified by the development shown in Fig. 25B, which analysis was carried out using the fluorescence intensity of CFSE as an index. Since the fluorescence signal of CFSE decreases as the cell division proceeds, the frequency of cell division can be identified based on the fluorescence intensity of CFSE. The brachial lymph node of the B16F10(-) anti-CD4 antibody(-) group and the B16F10(-) anti-CD4 antibody(+) group, and the tumor-draining brachial lymph node of the B16F10(+) anti-CD4 antibody(-) group and the B16F10(+) anti-CD4 antibody(+) group, were analyzed. Since the OT-1 and the Polyclonal CD8⁺ T cells did not show cell division in any of the groups and the growth of the Pmel-1 CD8⁺ T cells occurred only under the B16F10 tumor-bearing(+) conditions, it was suggested that the increase in the CD8⁺ T cells in the tumor-draining brachial lymph node due to the anti-CD4 antibody administration was tumor antigen-specific. In comparison between the B16F10(+) anti-CD4 antibody(-) group and the B16F10(+) anti-CD4 antibody(+) group, Pmel-1 CD8⁺ T cells that had undergone more frequent cell division were found in the B16F10(+) anti-CD4 antibody(+) group. It was therefore suggested that administration of the anti-CD4 antibody promotes the growth of tumor-specific CD8⁺ T cells.

Fig. 25D shows the percentage of cells with cell division frequency of 0 to 1, 2 to 4, and 5 to 8 in tumor-draining lymph node Pmel-1 CD8⁺ T cells from the B16F10(+) anti-CD4 antibody(-) group or the B16F10(+) anti-CD4 antibody(+) group. In the anti-CD4 antibody administration group, the percentage of cells that had undergone less frequent cell division, i.e. 0-1 and 2-4 times of cell division, was lower, while the percentage of cells that had undergone more frequent cell division, i.e. 5-8 times of cell division, was higher, than in the non-administration group with statistical significance (n=5, multiple t-tests; significance level: **, P<0.01; ***, P<0.001).

Fig. 25E shows the numbers of Pmel-1 CD8+ T cells with cell division frequency of 5 to 8 in peripheral blood (blood), tumor-draining lymph node (dLN), non-draining lymph node (ndLN), spleen (spleen), and tumor (tumor) in the B16F10(+) anti-CD4 antibody(-) group or the B16F10(+) anti-CD4 antibody(+) group. The cells with cell division frequency of 5 to 8 were mainly found in the tumor-draining lymph node, and the number of those cells was significantly larger in the anti-CD4 antibody administration group (αCD4) than in the non-administration group (Control) (n=5, multiple t-tests; significance level: *, P<0.05; **, P<0.01).

From the above results, it was strongly suggested that the CD8⁺ T cells that increase in the tumor-draining brachial lymph node when the anti-CD4 antibody is administered alone are tumor antigen-specific, and that such an increase is not a nonspecific increase (homeostatic proliferation) in T cells, which is often found under conditions where lymphocytes are decreased.
The following clauses detail embodiments of the invention.
1. A therapeutic agent for solid cancer, comprising as an effective ingredient an anti-CD4 antibody having a high cytotoxic activity, or an anti-CD4 antibody or antigen-binding fragment thereof which antibody or fragment comprises a cytotoxic component bound thereto, wherein said anti-CD4 antibody is a human-type chimeric antibody, humanized antibody or human antibody against human CD4.
2. The therapeutic agent according to clause 1, which comprises as an effective ingredient an anti-CD4 antibody having a high cytotoxic activity.
3. The therapeutic agent according to clause 1 or 2, wherein said solid cancer is solid cancer composed of spontaneously occurring cancer cells.
4. The therapeutic agent according to any one of clauses 1 to 3, wherein said solid cancer is epithelial solid cancer.
5. The therapeutic agent according to clause 4, wherein said epithelial solid cancer is at least one selected from the group consisting of colon cancer, lung cancer, pancreatic cancer, renal cancer, and breast cancer.
6. The therapeutic agent according to clause 4, wherein said epithelial solid cancer is at least one selected from the group consisting of colon cancer, lung cancer, pancreatic cancer, and renal cancer.
7. The therapeutic agent according to any one of clauses 1 to 3, wherein said solid cancer is at least one selected from melanoma and glioma.
8. The therapeutic agent according to any one of clauses 1 to 7, wherein said cytotoxic activity is ADCC activity.
9. The therapeutic agent according to any one of clauses 1 to 7, wherein said cytotoxic activity is CDC activity.
10. The therapeutic agent according to any one of clauses 1 to 9, which is for solid cancer at stage I to IV.
11. The therapeutic agent according to any one of clauses 1 to 10, which is used in combination with at least one selected from antagonists against an inhibitory immune checkpoint molecule, agonists against a co-stimulatory immune checkpoint molecule, substances having an action to stimulate cellular immunity or activate NK cells, immune cell therapy, and small molecule anticancer agents.
12. The therapeutic agent according to any one of clauses 1 to 10, which is used in combination with at least one selected from antagonists against an inhibitory immune checkpoint molecule, agonists against a co-stimulatory immune checkpoint molecule, substances having an action to stimulate cellular immunity or activate NK cells, and immune cell therapy.
13. The therapeutic agent according to clause 11 or 12, wherein said inhibitory immune checkpoint molecule is at least one selected from the group consisting of PD-1, CTLA-4, LAG-3, TIM-3, BTLA, PD-L1, PD-L2, CD80, CD86, GAL9, and HVEM, and said co-stimulatory immune checkpoint molecule is at least one selected from the group consisting of CD137, OX40, GITR, CD137L, OX40L, and TNFSF18.
14. The therapeutic agent according to clause 11 or 12, wherein said inhibitory immune checkpoint molecule is at least one selected from the group consisting of PD-1, CTLA-4, LAG-3, TIM-3, PD-L1, PD-L2, CD80, CD86, and GAL9, and said co-stimulatory immune checkpoint molecule is at least one selected from the group consisting of CD137, OX40, GITR, CD137L, and OX40L.
15. The therapeutic agent according to any one of clauses 11 to 14, wherein said antagonists and said agonists are antibodies against immune checkpoint molecule.
16. The therapeutic agent according to clause 15, wherein the antibody to be used in combination with said agent comprises at least one selected from an antagonistic anti-PD-1 antibody, an anti-PD-L1 antibody, and an anti-PD-L2 antibody.
17. The therapeutic agent according to clause 16, wherein the antibody to be used in combination with said agent further comprises at least one selected from the group consisting of an agonistic anti-CD137 antibody, an agonistic anti-OX40 antibody, and an antagonistic anti-CTLA-4 antibody.
18. The therapeutic agent according to clause 15, wherein the antibody to be used in combination with said agent comprises at least one selected from the group consisting of an antagonistic anti-PD-1 antibody, antagonistic anti-CTLA-4 antibody, antagonistic anti-LAG-3 antibody, antagonistic anti-TIM-3 antibody, antagonistic anti-BTLA antibody, anti-PD-L1 antibody, anti-PD-L2 antibody, agonistic anti-CD 137 antibody, agonistic anti-OX40 antibody, and an agonistic anti-GITR antibody.
19. The therapeutic agent according to clause 15, wherein the antibody to be used in combination with said agent comprises at least one selected from the group consisting of an antagonistic anti-PD-1 antibody, antagonistic anti-CTLA-4 antibody, anti-PD-L1 antibody, anti-PD-L2 antibody, agonisitc anti-CD 137 antibody, and an agonistic anti-OX40 antibody.
20. The therapeutic agent according to clause 15, wherein the antibody to be used in combination with said agent comprises an antagonistic anti-CTLA-4 antibody.
21. The therapeutic agent according to clause 15, wherein the antibody to be used in combination with said agent comprises at least one selected from the group consisting of an antagonistic anti-LAG-3 antibody, antagonistic anti-TIM-3 antibody, antagonistic anti-BTLA antibody, and an agonistic anti-GITR antibody.
22. The therapeutic agent according to any one of clauses 11 to 21, wherein said substance having an action to stimulate cellular immunity or activate NK cells is at least one selected from the group consisting of INF-α/□, IL-12, GM-CSF and chemokines.
23. The therapeutic agent according to any one of clauses 11 to 22, wherein said immune cell therapy is at least one selected from the group consisting of TIL therapy, LAK therapy, CTL therapy, and CAR-T therapy.
24. The therapeutic agent according to any one of clauses 1 to 23, which is for systemic administration.
25. A recurrence-suppressing agent for solid cancer, comprising as an effective ingredient an anti-CD4 antibody having a high cytotoxic activity, or an anti-CD4 antibody or antigen-binding fragment thereof which antibody or fragment comprises a cytotoxic component bound thereto, wherein said anti-CD4 antibody is a human-type chimeric antibody, humanized antibody or human antibody against human CD4.
26. The recurrence-suppressing agent according to clause 25, which is used in combination with at least one selected from antagonists against an inhibitory immune checkpoint molecule, agonists against a co-stimulatory immune checkpoint molecule, substances having an action to stimulate cellular immunity or activate NK cells, immune cell therapy, and small molecule anticancer agents.
27. The recurrence-suppressing agent according to clause 25, which is used in combination with at least one selected from antagonists against an inhibitory immune checkpoint molecule, agonists against a co-stimulatory immune checkpoint molecule, substances having an action to stimulate cellular immunity or activate NK cells, and immune cell therapy.
28. A metastasis-suppressing agent for solid cancer, comprising as an effective ingredient an anti-CD4 antibody having a high cytotoxic activity, or an anti-CD4 antibody or antigen-binding fragment thereof which antibody or fragment comprises a cytotoxic component bound thereto, wherein said anti-CD4 antibody is a human-type chimeric antibody, humanized antibody or human antibody against human CD4.
29. The metastasis-suppressing agent according to clause 28, which is used in combination with at least one selected from antagonists against an inhibitory immune checkpoint molecule, agonists against a co-stimulatory immune checkpoint molecule, substances having an action to stimulate cellular immunity or activate NK cells, immune cell therapy, and small molecule anticancer agents.
30. The metastasis-suppressing agent according to clause 28, which is used in combination with at least one selected from antagonists against an inhibitory immune checkpoint molecule, agonists against a co-stimulatory immune checkpoint molecule, substances having an action to stimulate cellular immunity or activate NK cells, and immune cell therapy.
31. An agent for enhancing activity of, promoting proliferation of, and/or promoting differentiation of CD8⁺ T cells specific to tumor antigen expressed by solid cancer in a solid cancer patient, and/or recruiting the CD8⁺ T cells specific to said tumor antigen to the tumor site in a solid cancer patient, said agent comprising as an effective ingredient an anti-CD4 antibody having a high cytotoxic activity, or an anti-CD4 antibody or antigen-binding fragment thereof which antibody or fragment comprises a cytotoxic component bound thereto, wherein said anti-CD4 antibody is a human-type chimeric antibody, humanized antibody or human antibody against human CD4.
32. A method of treating solid cancer, comprising administering an effective amount of an anti-CD4 antibody having a high cytotoxic activity, or an anti-CD4 antibody or antigen-binding fragment thereof which antibody or fragment comprises a cytotoxic component bound thereto, to a patient in need of treating solid cancer, wherein said anti-CD4 antibody is a human-type chimeric antibody, humanized antibody or human antibody against human CD4.
33. A method of suppressing recurrence of solid cancer, comprising administering an effective amount of an anti-CD4 antibody having a high cytotoxic activity, or an anti-CD4 antibody or antigen-binding fragment thereof which antibody or fragment comprises a cytotoxic component bound thereto, to a patient in need of suppressing recurrence of solid cancer, wherein said anti-CD4 antibody is a human-type chimeric antibody, humanized antibody or human antibody against human CD4.
34. A method of suppressing metastasis of solid cancer, comprising administering an effective amount of an anti-CD4 antibody having a high cytotoxic activity, or an anti-CD4 antibody or antigen-binding fragment thereof which antibody or fragment comprises a cytotoxic component bound thereto, to a patient in need of suppressing metastasis of solid cancer, wherein said anti-CD4 antibody is a human-type chimeric antibody, humanized antibody or human antibody against human CD4.
35. A method of enhancing activity of, promoting proliferation of, and/or promoting differentiation of CD8⁺ T cells specific to tumor antigen expressed by solid cancer in a solid cancer patient, and/or recruiting the CD8⁺ T cells specific to said tumor antigen to the tumor site in a solid cancer patient, said method comprising administering an effective amount of an anti-CD4 antibody having a high cytotoxic activity, or an anti-CD4 antibody or antigen-binding fragment thereof which antibody or fragment comprises a cytotoxic component bound thereto, to said patient, wherein said anti-CD4 antibody is a human-type chimeric antibody, humanized antibody or human antibody against human CD4.

## Claims

1. An agent for use in treatment of solid cancer, comprising as an effective ingredient an anti-CD4 antibody having a high cytotoxic activity, or an anti-CD4 antibody or antigen-binding fragment thereof which antibody or fragment comprises a cytotoxic component bound thereto, preferably comprising as an effective ingredient an anti-CD4 antibody having a high cytotoxic activity, wherein said anti-CD4 antibody is a human-type chimeric antibody, humanized antibody or human antibody against human CD4, and wherein said agent is for use in combination with an antagonistic anti-LAG-3 antibody.

2. The agent for use according to claim 1, wherein said solid cancer is solid cancer composed of spontaneously occurring cancer cells.

3. The agent for use according to claim 1 or 2, wherein said solid cancer is epithelial solid cancer, optionally wherein said epithelial solid cancer is at least one selected from the group consisting of colon cancer, lung cancer, pancreatic cancer, renal cancer, and breast cancer, or at least one selected from the group consisting of colon cancer, lung cancer, pancreatic cancer, and renal cancer.

4. The agent for use according to claim 1 or 2, wherein said solid cancer is at least one selected from melanoma and glioma.

5. The agent for use according to any one of claims 1 to 4, wherein said cytotoxic activity is ADCC activity.

6. The agent for use according to any one of claims 1 to 4, wherein said cytotoxic activity is CDC activity.

7. The agent for use according to any one of claims 1 to 6, which is for use in treatment of solid cancer at stage I to IV.

8. The agent for use according to any one of claims 1 to 7, which is for systemic administration.

9. The agent for use according to any one of claims 1 to 8, wherein the anti-CD4 antibody having a high cytotoxic activity has an ADCC activity that is 100 times higher than the ADCC activity of the known anti-CD4 antibody 6G5 and/or CE9.1.

10. The agent for use according to any one of claims 1 to8, wherein the anti-CD4 antibody having a high cytotoxic activity has an ADCC activity and shows the ADCC activity at a concentration of 0.001 µg/mL.

11. The agent for use according to any one of claims 1 to 8, wherein the anti-CD4 antibody having a high cytotoxic activity has an ADCC activity and shows the ADCC activity at a concentration of 0.01 µg/mL.

12. An agent for use in suppression of recurrence of solid cancer, comprising as an effective ingredient an anti-CD4 antibody having a high cytotoxic activity, or an anti-CD4 antibody or antigen-binding fragment thereof which antibody or fragment comprises a cytotoxic component bound thereto, wherein said anti-CD4 antibody is a human-type chimeric antibody, humanized antibody or human antibody against human CD4, and wherein said agent is for use in combination with an antagonistic anti-LAG-3 antibody.

13. An agent for use in suppression of metastasis of solid cancer, comprising as an effective ingredient an anti-CD4 antibody having a high cytotoxic activity, or an anti-CD4 antibody or antigen-binding fragment thereof which antibody or fragment comprises a cytotoxic component bound thereto, wherein said anti-CD4 antibody is a human-type chimeric antibody, humanized antibody or human antibody against human CD4, and wherein said agent is for use in combination with an antagonistic anti-LAG-3 antibody.

14. An agent for use in enhancing activity of, promoting proliferation of, and/or promoting differentiation of CD8⁺ T cells specific to tumor antigen expressed by solid cancer in a solid cancer patient, and/or recruiting the CD8⁺ T cells specific to said tumor antigen to the tumor site in a solid cancer patient, said agent comprising as an effective ingredient an anti-CD4 antibody having a high cytotoxic activity, or an anti-CD4 antibody or antigen-binding fragment thereof which antibody or fragment comprises a cytotoxic component bound thereto, wherein said anti-CD4 antibody is a human-type chimeric antibody, humanized antibody or human antibody against human CD4, and wherein said agent is for use in combination with an antagonistic anti-LAG-3 antibody.
